# EUROPÄISCHE PATENTSCHRIFT

(11) **EP 0 638 563 B1**
(45) Veröffentlichungstag und Bekanntmachung des Hinweises auf die Patenterteilung: **15.10.1997**
(21) Anmeldenummer: 94111711.1
(22) Anmeldetag: 27.07.1994
(51) Int. Cl.: C07D 265/06, C07D 265/12, C07D 265/26, A01N 43/86

(54) **Aminoaryl-1,3-oxazin-2,4-dione**
Aminoaryl-1,3-oxazin-2,4-diones
Aminoaryl-1,3-oxazin-2,4-diones

(30) Priorität: 09.08.1993 DE 4326649
(43) Veröffentlichungstag der Anmeldung: 15.02.1995
(73) Patentinhaber: BAYER AG, 51368 Leverkusen (DE)
(72) Erfinder: Ooms, Dr. Pieter, D-47800 Krefeld (DE); Santel, Dr. Hans-Joachim, D-51371 Leverkusen (DE); Dollinger, Dr. Markus, D-51381 Leverkusen (DE)

(56) Entgegenhaltungen:
- EP-A- 0 371 240
- EP-A- 0 529 402
- DE-A- 2 260 859

## Beschreibung

Die Erfindung betrifft neue Aminoaryl-1,3-oxazin-2,4-dione, Verfahren zu ihrer Herstellung, neue Zwischenprodukte und ihre Verwendung als Herbizide.

Es ist bereits bekannt, daß bestimmte N-Aryl-1,3-oxazin-2,4-dione, wie z.B. 3-(2-Fluor-4-chlor-5-chlordifluormethoxy-phenyl)-6,7-dihydro-cyclopent[e]-1,3-oxazin-2,4-(3H,5H)-dion, herbizide Eigenschaften aufweisen (vgl. EP-A 371240; vgl. auch EP-A 529402).

Die herbizide Wirkung dieser bekannten Verbindungen ist jedoch nicht in allen Belangen zufriedenstellend.

Es wurden nun neue Aminoaryl-1,3-oxazin-2,4-dione der allgemeinen Formel (I) gefunden, 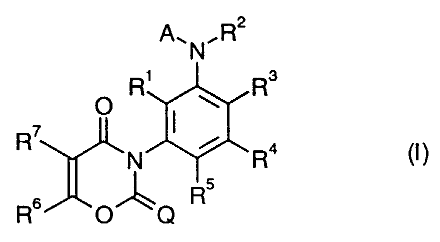 in welcher
- A: für Wasserstoff oder C₁-C₆-Alkyl steht,
- Q: für Sauerstoff oder Schwefel steht,
- R¹: für Wasserstoff oder Halogen steht,
- R²: für Wasserstoff, für einen jeweils gegebenenfalls durch Halogen oder C₁-C₄-Alkoxy substituierten Rest der Reihe C₁-C₆-Alkyl, C₂-C₆-Alkenyl, C₂-C₆-Alkinyl, C₁-C₆-Alkyl-carbonyl, C₁-C₆-Alkoxy-carbonyl, C₁-C₆-Alkyl-amino-carbonyl, C₁-C₆-Alkyl-sulfonyl oder für Di-(C₁ -C₄-alkyl)-amino-sulfonyl steht,
- R²: weiterhin für einen jeweils gegebenenfalls durch Halogen, Cyano, Nitro, Carboxy, Carbamoyl, C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) oder durch C₁-C₄-Alkoxy-carbonyl substituierten Rest der Reihe Phenyl, Phenylcarbonyl, Phenyl-C₁-C₄-alkyl, Phenylsulfonyl oder Phenyl-C₁-C₄-alkylsulfonyl steht,
- R³: für Wasserstoff, Halogen, Cyano, Methyl, Trifluormethyl oder Methoxycarbonyl steht,
- R⁴: für Wasserstoff oder Halogen steht,
- R⁵: für Wasserstoff oder Halogen steht,
- R⁶: für Wasserstoff, Halogen oder für jeweils gegebenenfalls durch Halogen substituiertes C₁-C₄-Alkyl oder Phenyl steht und
- R⁷: für Wasserstoff, Halogen oder für jeweils gegebenenfalls durch Halogen substituiertes C₁-C₄-Alkyl oder Phenyl steht, oder zusammen mit R⁶ für C₃-C₅-Alkandiyl steht, oder zusammen mit R⁶ eine Benzogruppierung bildet.

Man erhält die neuen Aminoaryl-1,3-oxazin-2,4-dione der allgemeinen Formel (I), wenn man
(a) Nitroaryl-1,3-oxazin-2,4-dione der allgemeinen Formel (II) 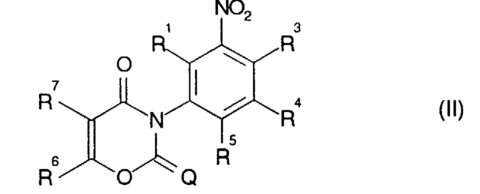 in welcher
   Q, R¹, R³, R⁴, R⁵, R⁶ und R⁷ die oben angegebenen Bedeutungen haben,
   mit einem Reduktionsmittel, gegebenenfalls in Gegenwart eines Katalysators oder Reaktionshilfsmittels und gegebenenfalls in Gegenwart eines Verdünnungsmittels umsetzt, oder wenn man
(b) Aminoaryl-1,3-oxazin-2,4-dione der allgemeinen Formel (Ia) 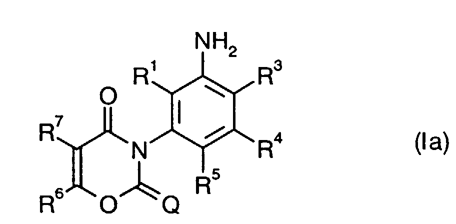 in welcher
   Q, R¹, R³, R⁴, R⁵, R⁶ und R⁷ die oben angegebenen Bedeutungen haben,
   mit elektrophilen Verbindungen der allgemeinen Formel (III)

   X¹-A¹ (III)

   in welcher
   - A¹: für C₁-C₆-Alkyl steht und
   - X¹: für Halogen oder die Gruppierung -O-SO₂-O-A¹ steht,
und/oder mit elektrophilen Verbindungen der allgemeinen Formel (IV)

X²-R² (IV)

in welcher
- R²: mit Ausnahme von Wasserstoff die oben angegebene Bedeutung hat und
- X²: für Halogen oder die Gruppierung 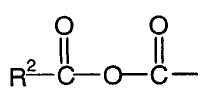 steht, und/oder gegebenenfalls mit Isocyanaten der allgemeinen Formel (V)

O=C=N-R⁸ (V)

in welcher
R⁸ für C₁-C₆-Alkyl steht,
gegebenenfalls in Gegenwart eines Säureakzeptors und gegebenenfalls in Gegenwart eines Verdünnungsmittels umsetzt, wobei gegebenenfalls die Reihenfolge der Umsetzung mit den Verbindungen der Formeln (III) und/oder (IV) und/oder (V) auch anders als oben angegeben sein kann.

Die neuen Aminoaryl-1,3-oxazin-2,4-dione der allgemeinen Formel (I) zeichnen sich durch starke herbizide Wirksamkeit aus.

Die Erfindung betrifft vorzugsweise Verbindungen der Formel (I), in welcher
- A: für Wasserstoff oder C₁-C₄-Alkyl steht,
- Q: für Sauerstoff oder Schwefel steht,
- R¹: für Wasserstoff, Fluor, Chlor oder Brom steht,
- R²: für Wasserstoff, für einen jeweils gegebenenfalls durch Fluor, Chlor, Methoxy oder Ethoxy substituierten Rest der Reihe C₁-C₄-Alkyl, C₂-C₄-Alkenyl, C₂-C₄-Alkinyl, C₁-C₄-Alkyl-carbonyl, C₁-C₄-Alkoxy-carbonyl, C₁-C₄-Alkylamino-carbonyl oder C₁-C₄-Alkyl-sulfonyl oder Di-(C₁-C₃-alkyl)amino-sulfonyl steht,
- R²: weiterhin für einen jeweils gegebenenfalls durch Halogen, Cyano, Nitro, Carboxy, Carbamoyl, C₁-C₃-Alkyl, C₁-C₃-Alkoxy, C₁-C₃-Alkylthio, C₁-C₃-Alkylsulfinyl, C₁-C₃-Alkylsulfonyl (welche jeweils gegebenenfalls durch Fluor und/oder Chlor substituiert sind) oder C₁-C₃-Alkoxy-carbonyl substituierten Rest der Reihe Phenyl, Phenylcarbonyl, Phenyl-C₁-C₃-alkyl, Phenylsulfonyl oder Phenyl-C₁-C₃-alkylsulfonyl steht,
- R³: für Wasserstoff, Fluor, Chlor, Brom, Cyano, Methyl, Trifluormethyl oder Methoxycarbonyl steht,
- R⁴: für Wasserstoff, Fluor, Chlor oder Brom steht,
- R⁵: für Wasserstoff, Fluor, Chlor oder Brom steht,
- R⁶: für Wasserstoff, Fluor, Chlor, Brom oder für jeweils gegebenenfalls durch Fluor und/oder Chlor substituiertes C₁-C₃-Alkyl oder Phenyl steht und
- R⁷: für Wasserstoff, Fluor, Chlor, Brom oder für jeweils gegebenenfalls durch Fluor und/oder Chlor substituiertes C₁-C₃-Alkyl oder Phenyl steht, oder zusammen mit R⁶ für C₃-C₄-Alkandiyl steht, oder zusammen mit R⁶ eine Benzogruppierung bildet.

Die in den Substituentendefinitionen genannten gesättigten oder ungesättigten Kohlenwasserstoffreste wie Alkyl, Alkenyl, Alkinyl sind - auch in Verbindungen mit Heteroatomen wie in Alkoxy, Alkylthio etc. - jeweils geradkettig oder verzweigt.

Halogen steht im allgemeinen für Fluor, Chlor, Brom oder Iod, vorzugsweise für Fluor, Chlor oder Brom.

Die Erfindung betrifft insbesondere Verbindungen der Formel (I), in welcher
- A: für Wasserstoff, Methyl, Ethyl, n- oder i-Propyl steht,
- Q: für Sauerstoff oder Schwefel steht,
- R¹: für Wasserstoff steht,
- R²: für Wasserstoff, Methyl, Ethyl, n- oder i-Propyl, Allyl, Propargyl, für jeweils gegebenenfalls durch Fluor oder Chlor substituiertes Acetyl oder Propionyl, für Methoxycarbonyl, Ethoxycarbonyl, Methylaminocarbonyl oder Ethylaminocarbonyl, für jeweils gegebenenfalls durch Fluor oder Chlor substituiertes Methylsulfonyl oder Ethylsulfonyl, für Dimethylaminosulfonyl, für jeweils gegebenenfalls durch Fluor, Chlor, Cyano, Nitro, Carboxy, Methyl, Trifluormethyl, Methoxy oder Methoxycarbonyl substituiertes Phenyl, Benzoyl, Benzyl, Phenylsulfonyl oder Phenylmethylsulfonyl steht,
- R³: für Chlor, Brom oder Cyano steht,
- R⁴: für Wasserstoff steht,
- R⁵: für Wasserstoff, Chlor oder Fluor steht,
- R⁶: für Wasserstoff, Chlor, Brom, Methyl oder Trifluormethyl steht und
- R⁷: für Wasserstoff, Chlor, Brom, Methyl oder Trifluormethyl steht, oder zusammen mit R⁶ für Trimethylen oder Tetramethylen steht.

Beispiele für die erfindungsgemäßen Verbindungen der Formel (I) sind in der nachstehenden Tabelle 1 aufgeführt.

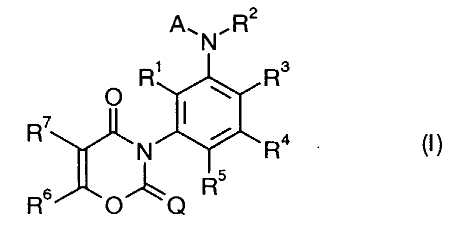

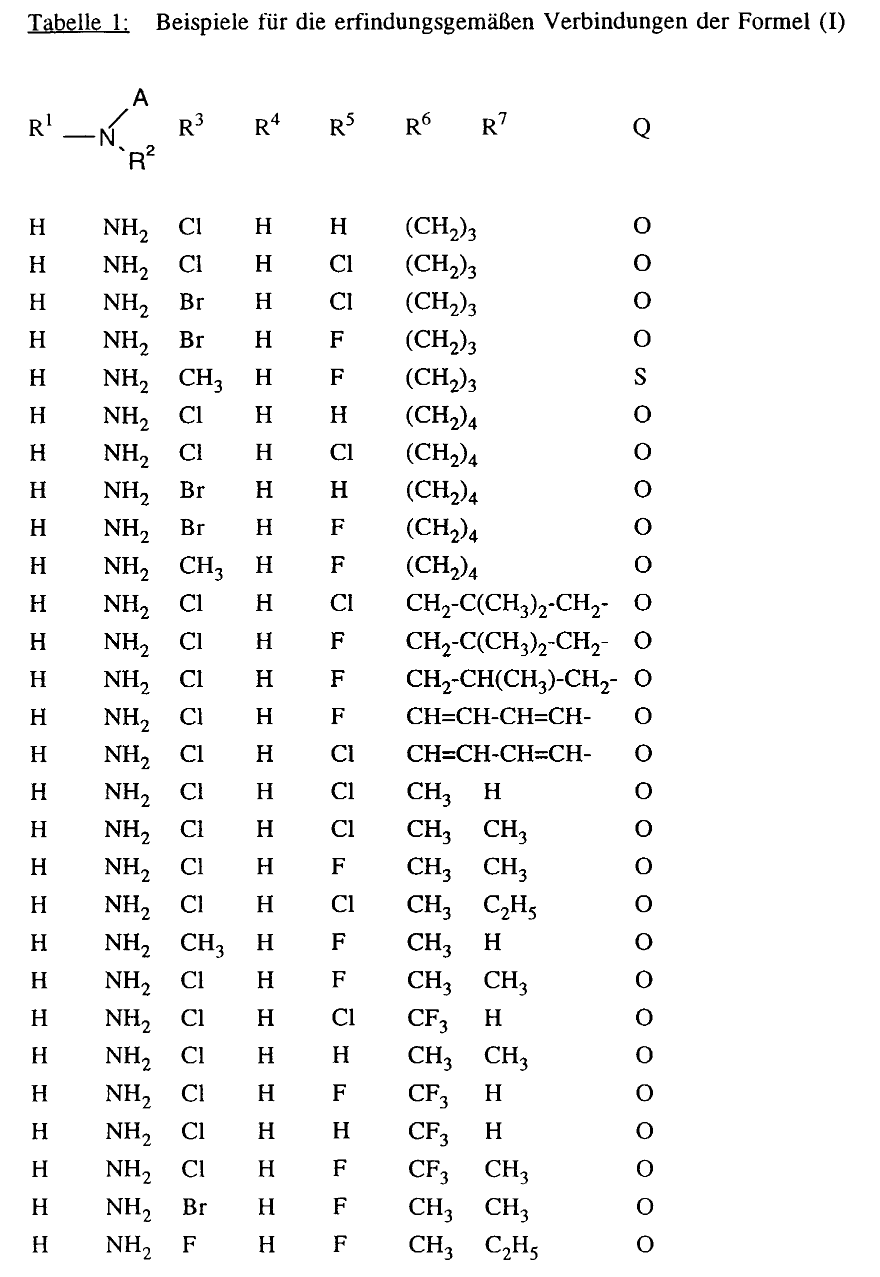

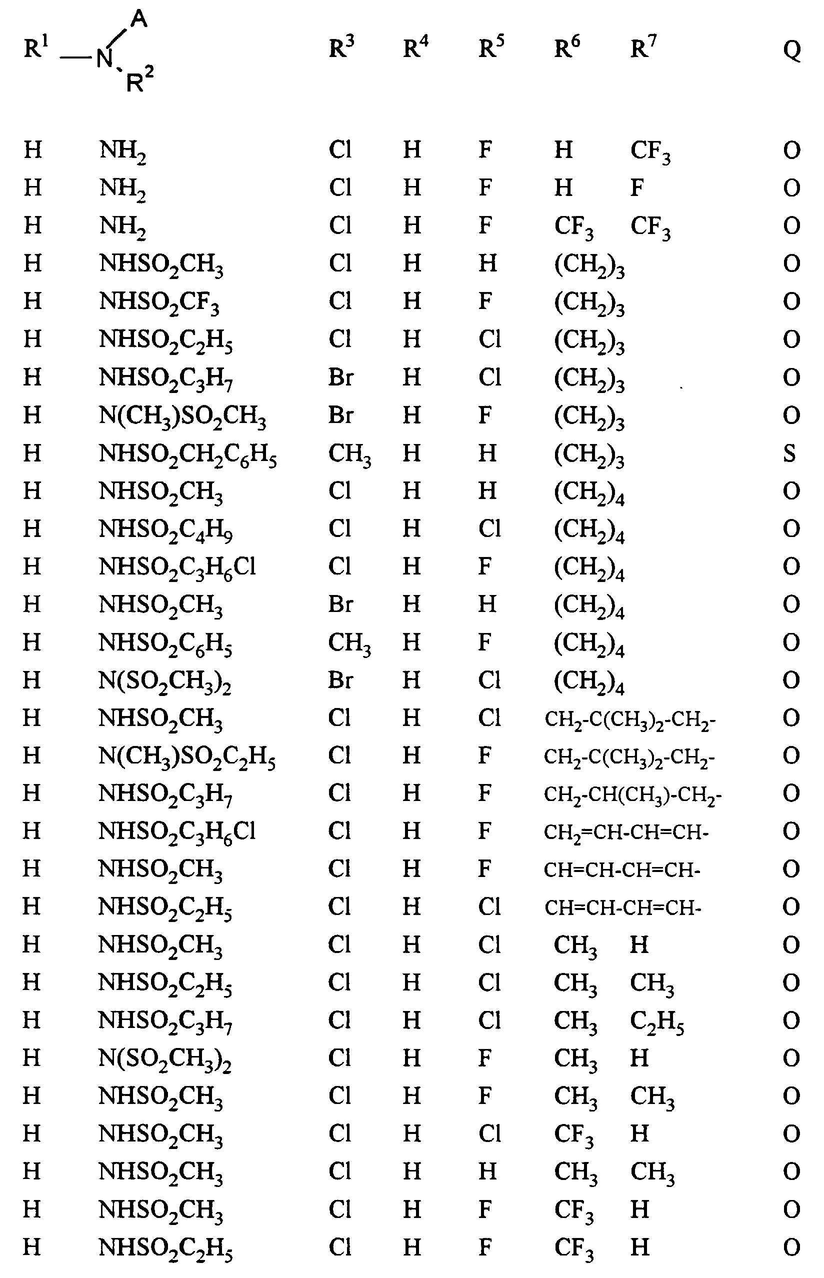

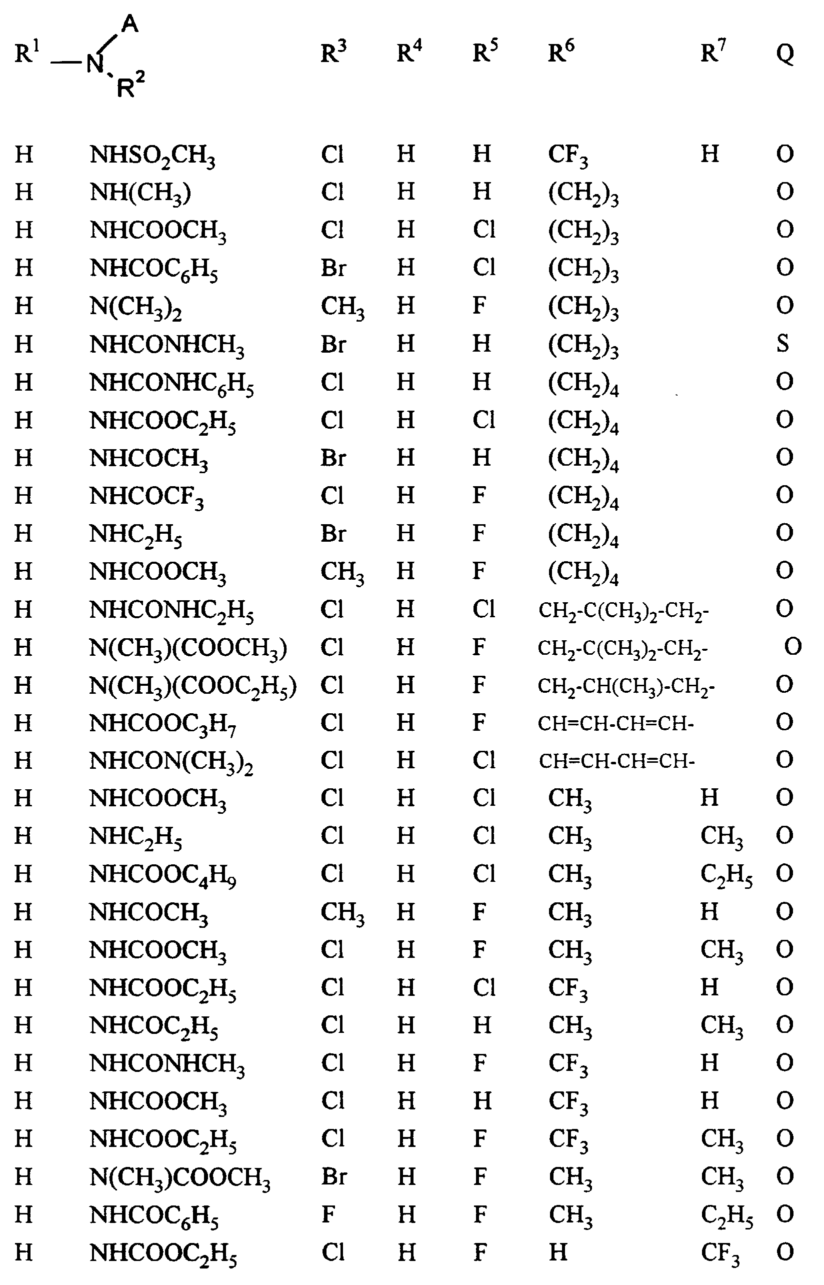

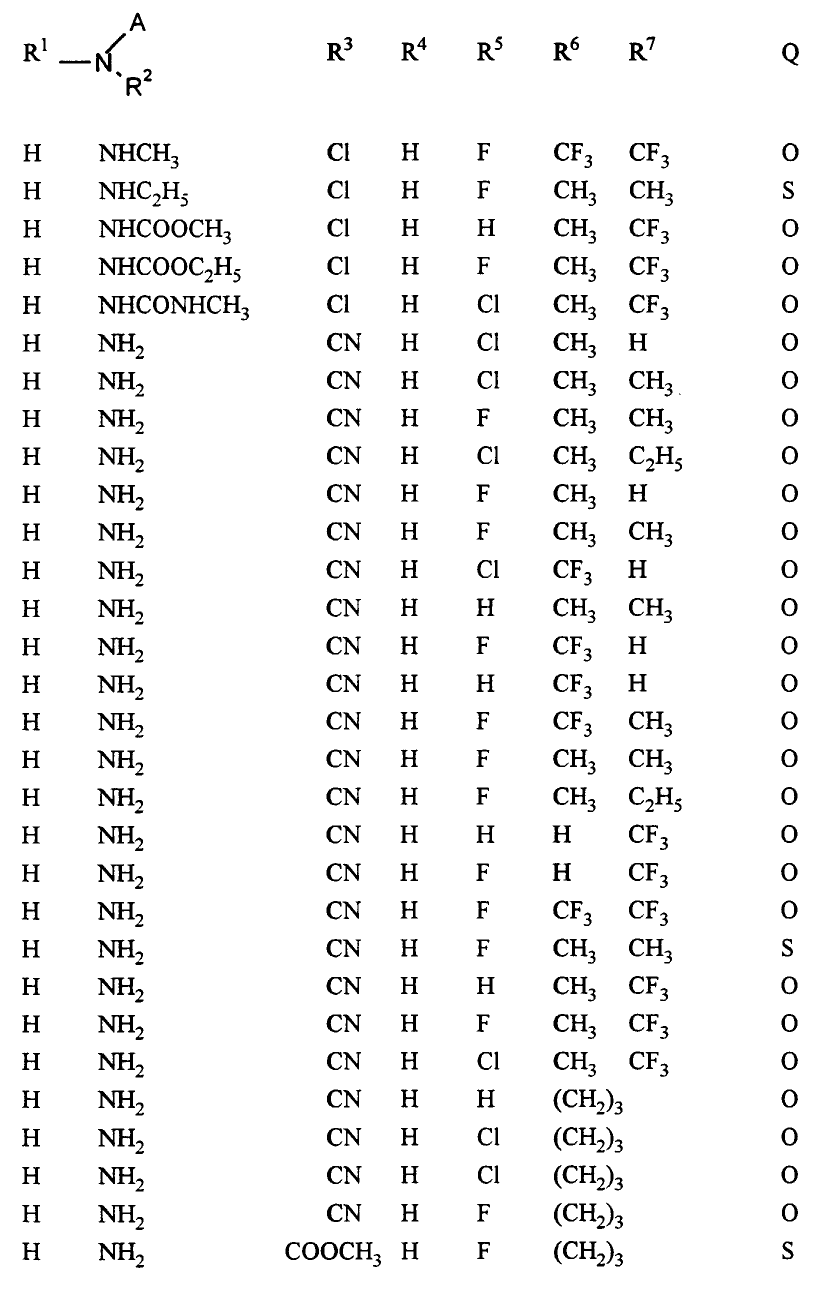

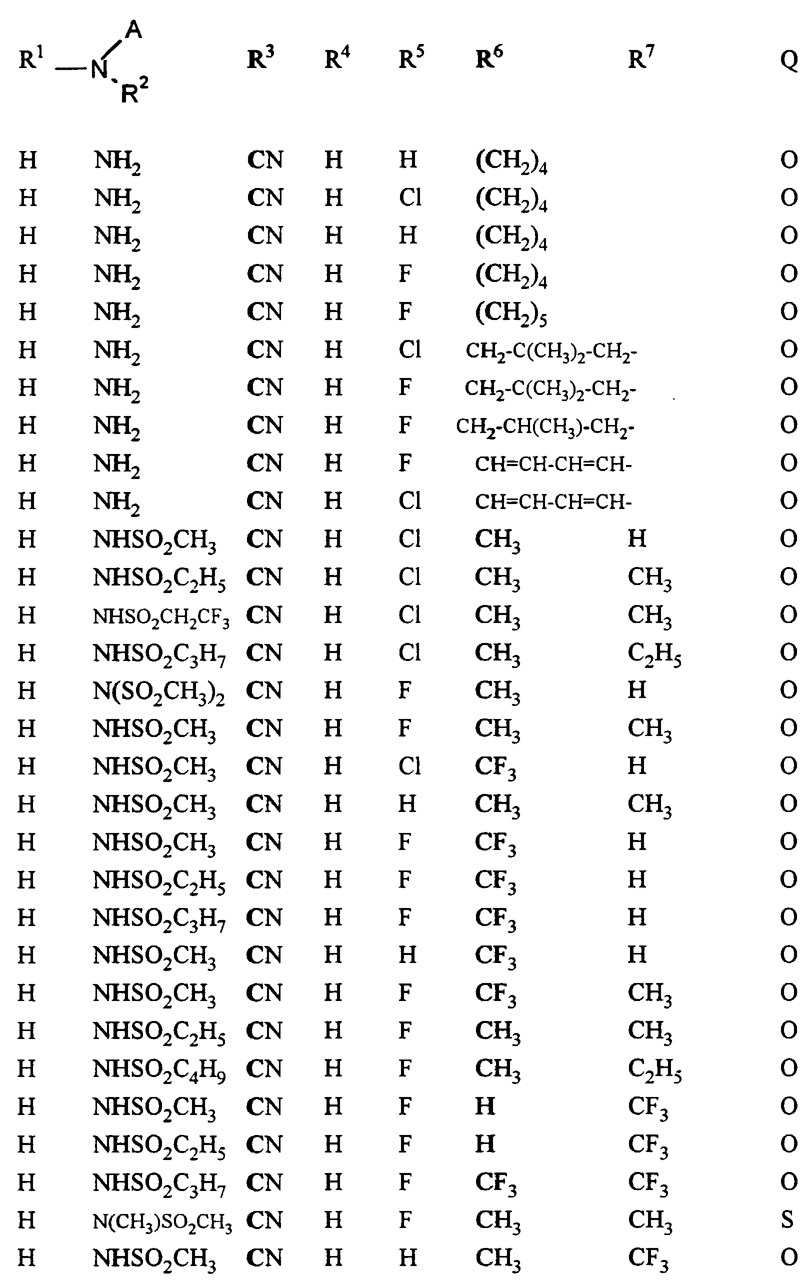

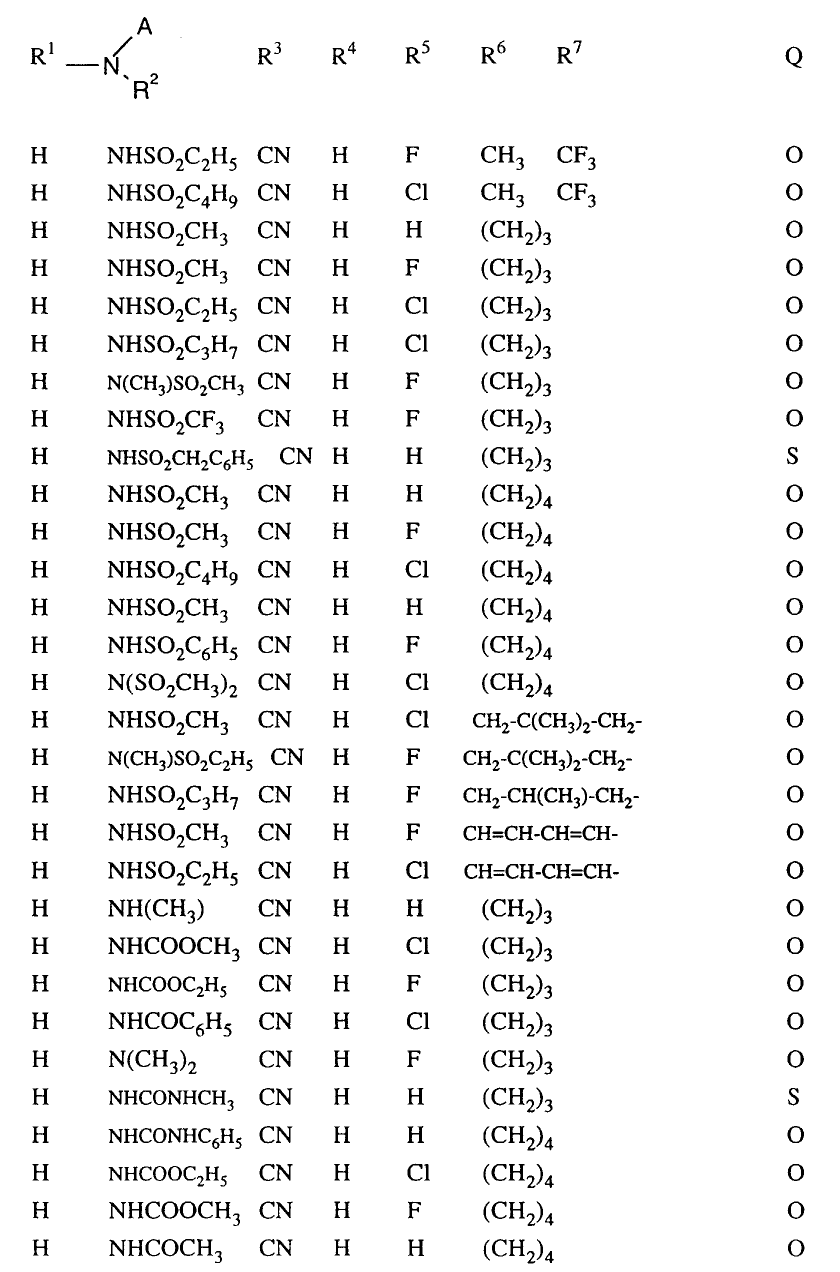

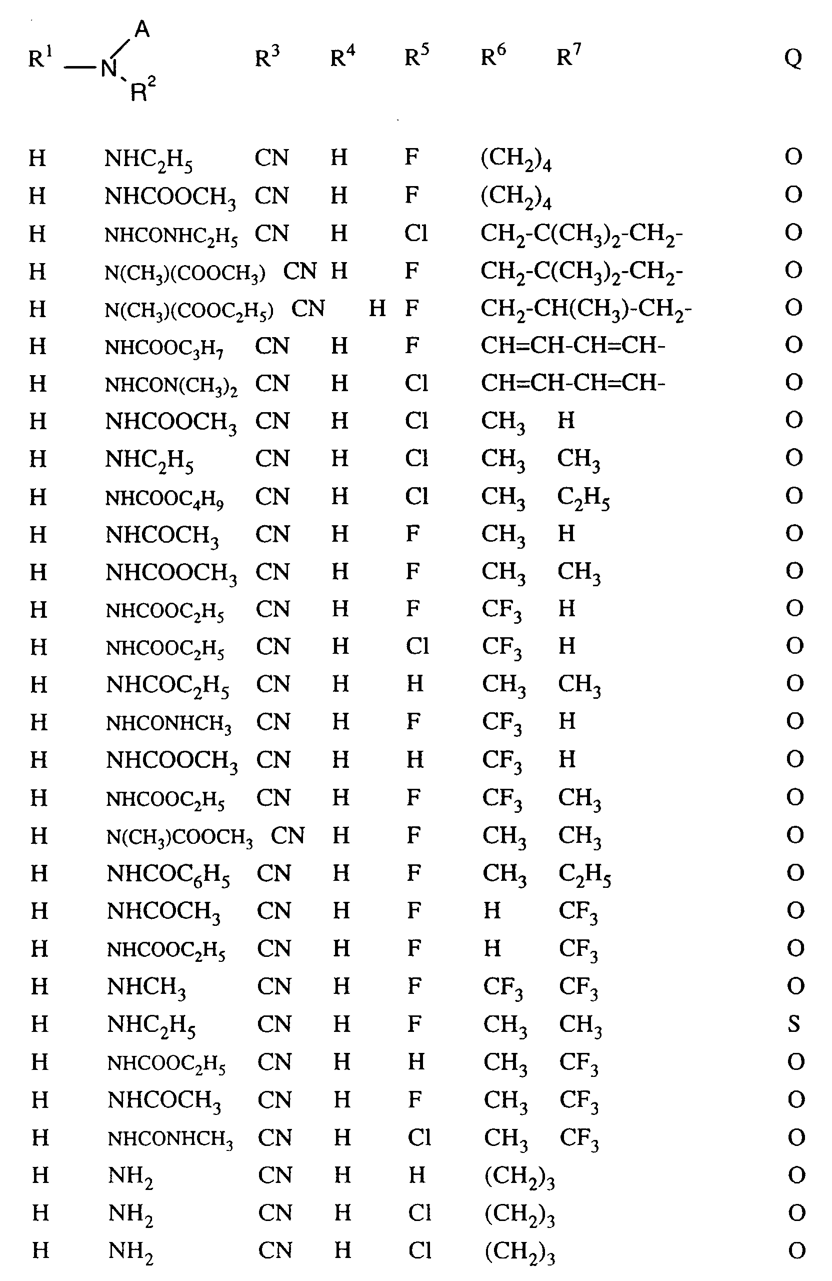

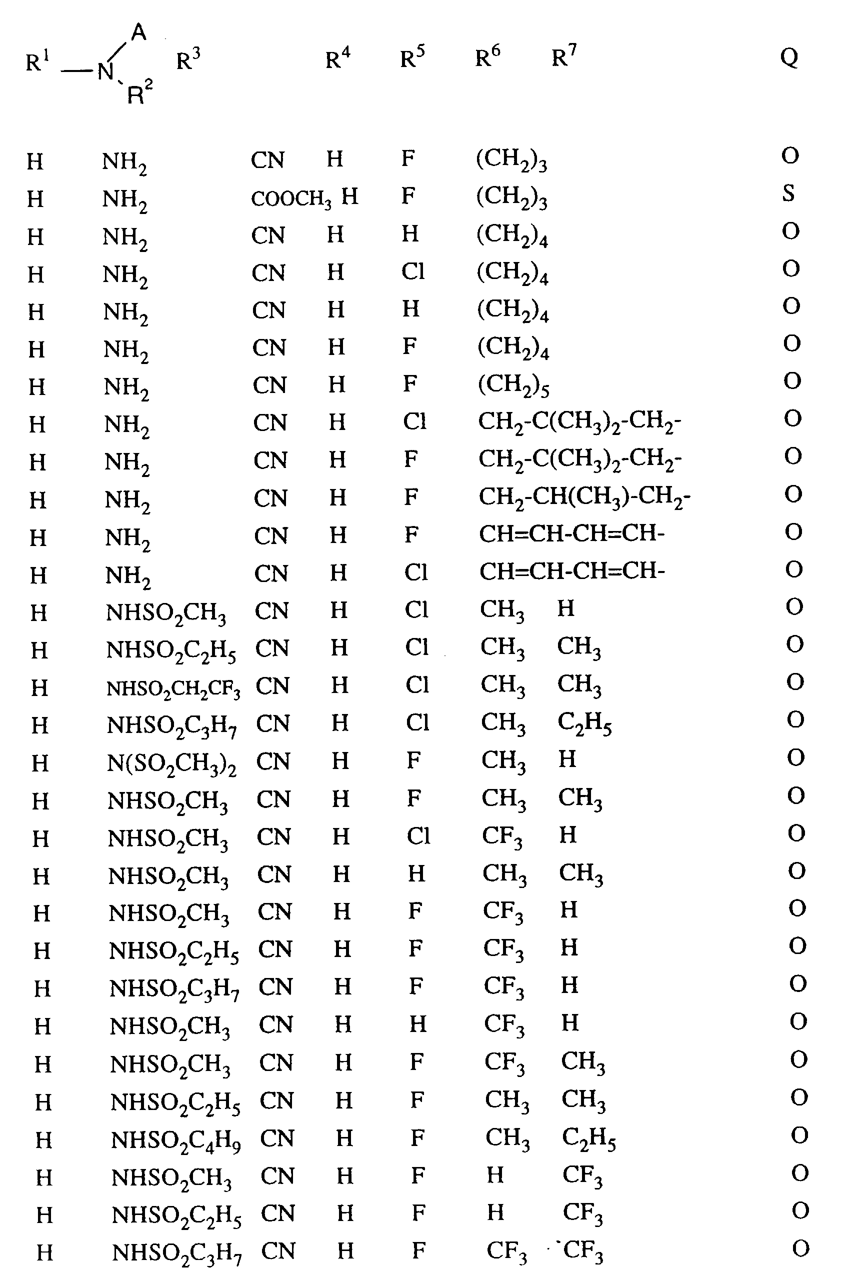

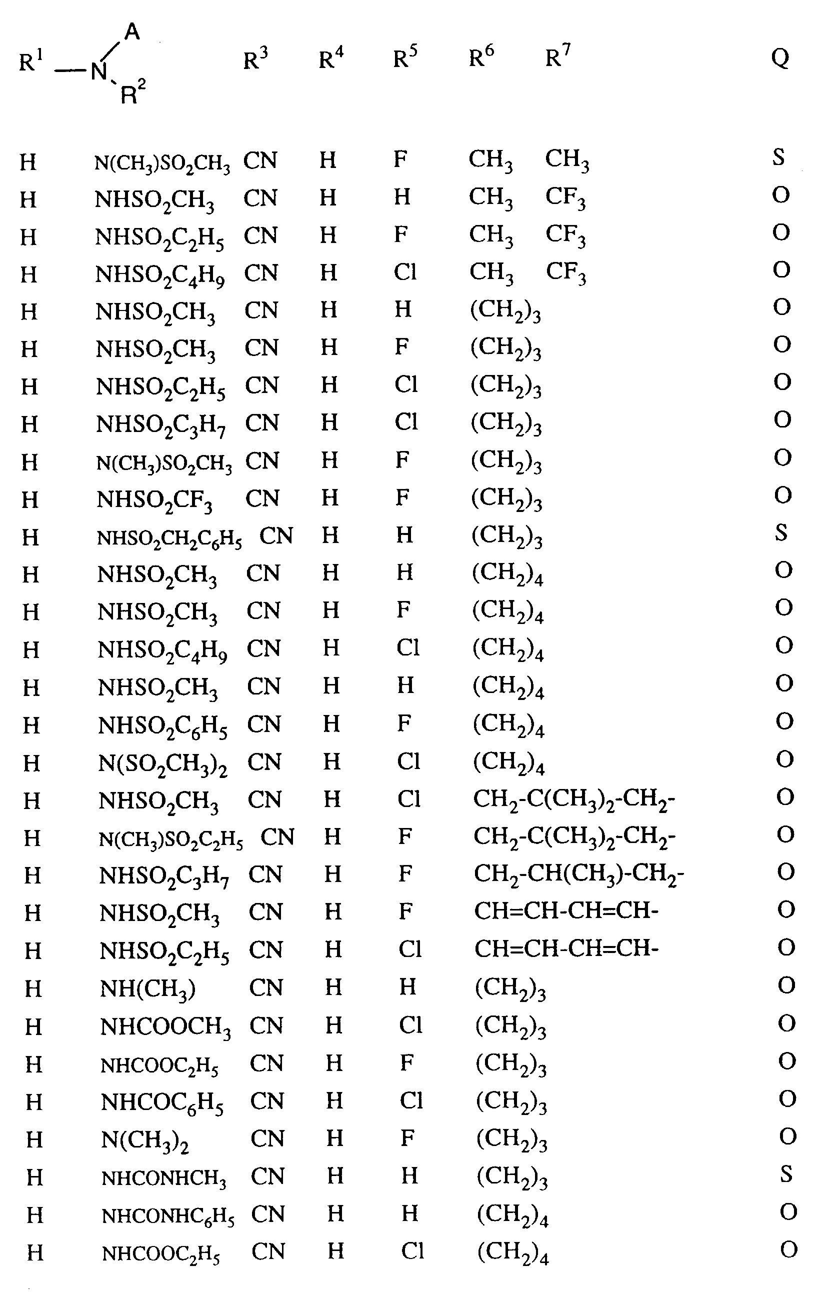

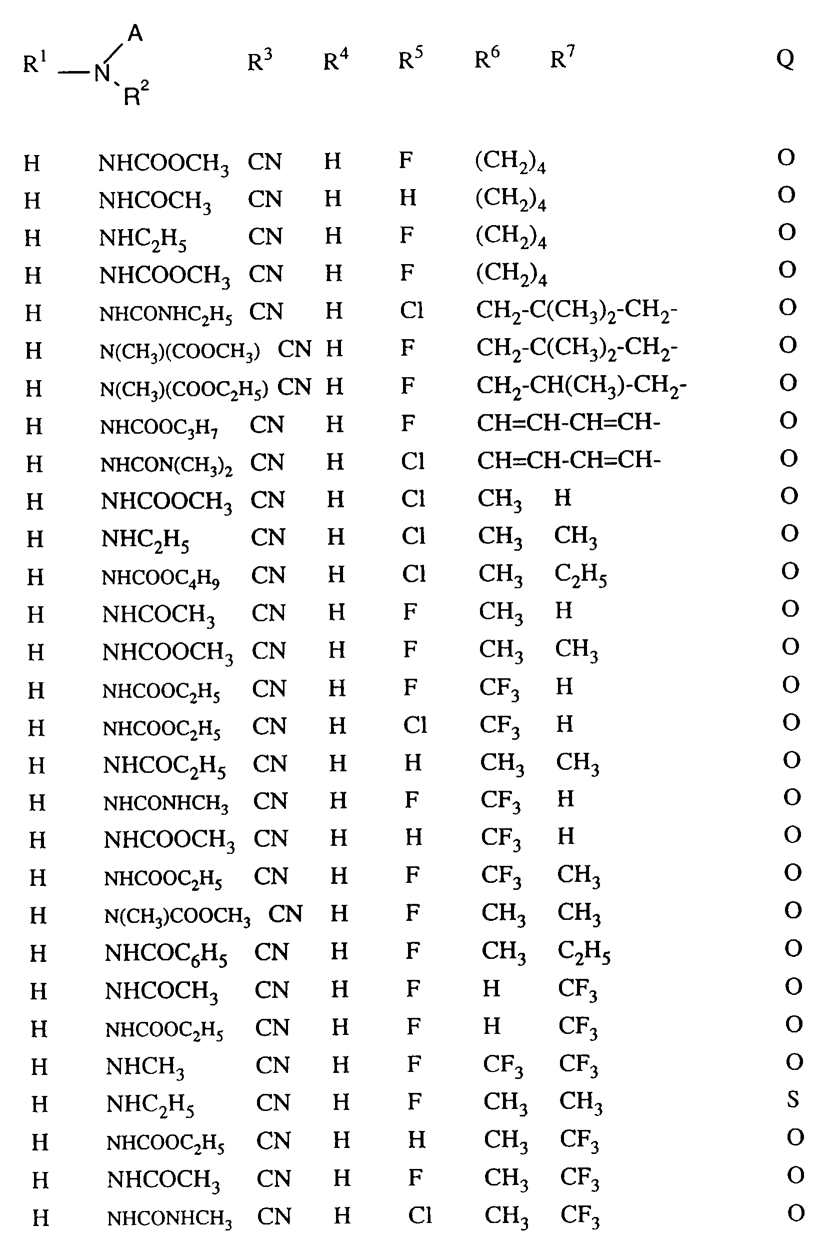

Verwendet man beim erfindungsgemäßen Verfahren (a) beispielsweise 3-(4-Chlor-2-fluor-5-nitrophenyl)-6-methyl-5-trifluormethyl-1,3-oxazin-2,4(3H,5H)-dion und Eisen in Gegenwart von Essigsäure als Ausgangsstoffe, so kann der Reaktionsverlauf durch das folgende Formelschema wiedergegeben werden:

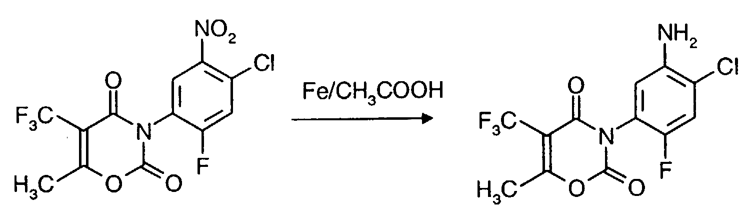

Verwendet man beim erfindungsgemäßen Verfahren (a) beispielsweise 3-(4-Brom-2-fluor-5-nitrophenyl)-benzo[e]-1,3-oxazin-2,4(3H,5H)-dion und Wasserstoff als Ausgangsstoffe, so kann der Reaktionsverlauf durch das folgende Formelschema wiedergegeben werden:

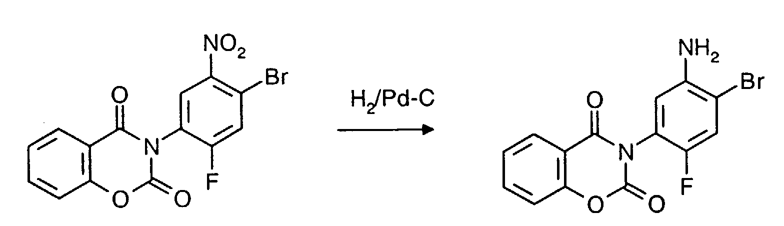

Verwendet man beim erfindungsgemäßen Verfahren (b) beispielsweise 3-(5-Amino-2-chlor-4-cyano-phenyl)-5-chlor-6-methyl-1,3-oxazin-2,4(3H,5H)-dion und Butansulfonsäurechlorid als Ausgangsstoffe, so kann der Reaktionsverlauf durch das folgende Formelschema wiedergegeben werden:

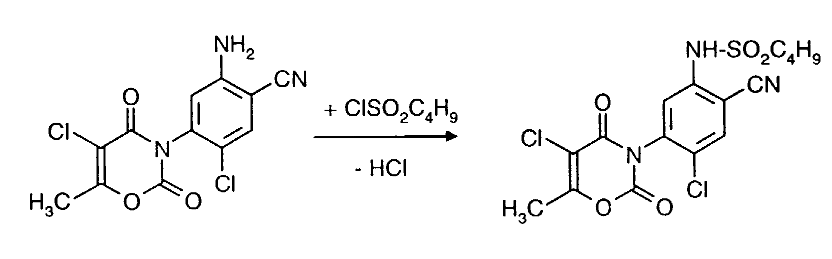

Die beim erfindungsgemäßen Verfahren (a) zur Herstellung von Verbindungen der Formel (I) als Ausgangsstoffe zu verwendenden Nitroaryl-1,3-oxazin-2,4-dione sind durch die Formel (II) allgemein definiert.

In Formel (II) haben Q, R¹, R³, R⁴, R⁵, R⁶ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R¹, R³, R⁴, R⁵, R⁶ und R⁷ angegeben wurden.

Beispiele für die Ausgangsstoffe der Formel (II) sind in der nachstehenden Tabelle 2 aufgeführt.

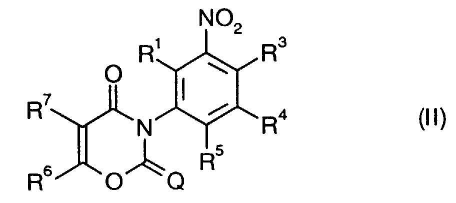

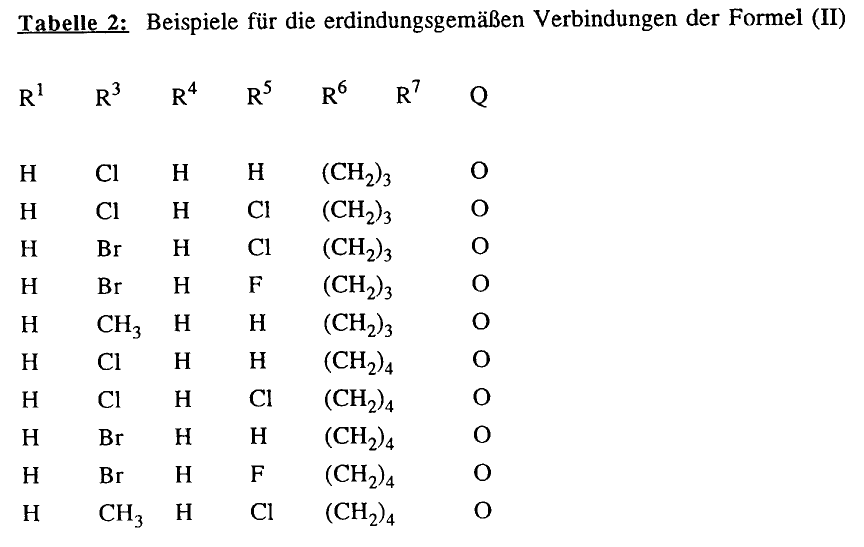

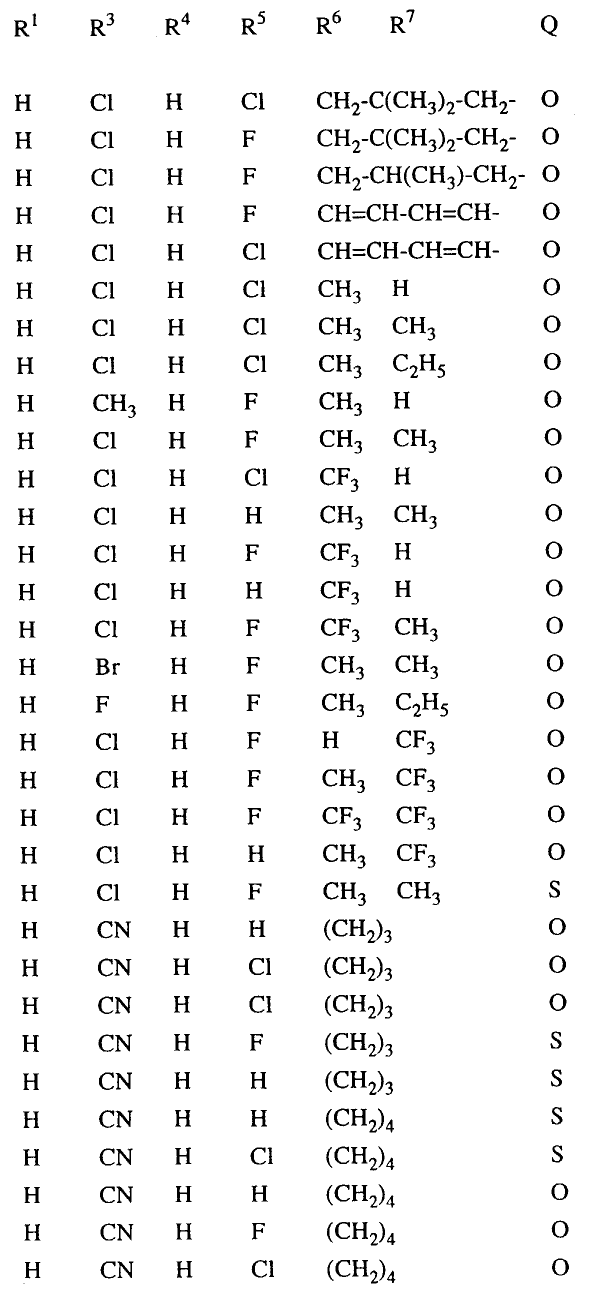

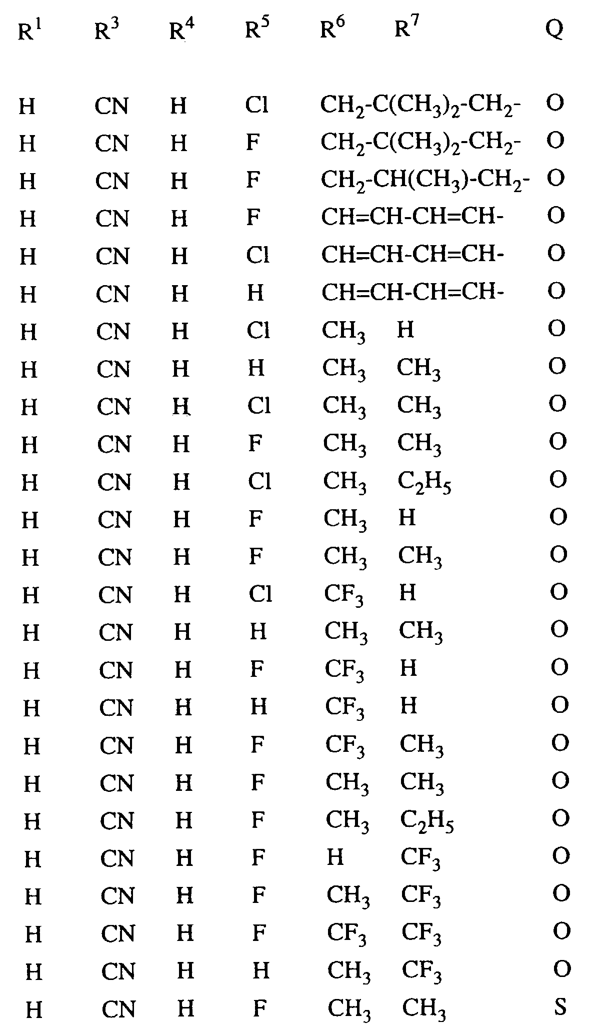

Die Nitroaryl-1,3-oxazin-2,4-dione der Formel (II) sind noch nicht aus der Literatur bekannt; sie sind als neue Stoffe Gegenstand der vorliegenden Patentanmeldung.

Man erhält die neuen Verbindungen der Formel (II), wenn man Aryliso(thio)cyanate der allgemeinen Formel (VI) 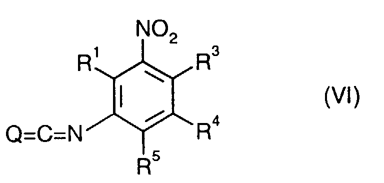 in welcher
Q, R¹, R³, R⁴ und R⁵ die oben angegebenen Bedeutungen haben,
(α) mit 1,3-Dioxin-4-onen der allgemeinen Formel (VII) 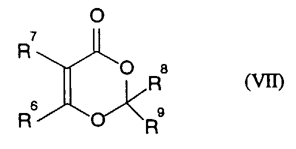 in welcher
   R⁶ und R⁷ die oben angegebenen Bedeutungen haben und
   R⁸ und R⁹ für Wasserstoff oder gegebenenfalls substituiertes Alkyl stehen,
   gegebenenfalls in Gegenwart eines Verdünnungsmittels umsetzt, oder
(β) mit Dicarbonsäuredichloriden der allgemeinen Formel (VIII) 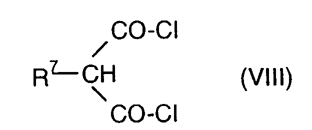 in welcher
   - R⁷: die oben angegebene Bedeutung hat,
   gegebenenfalls in Gegenwart eines Verdünnungsmittels umsetzt, oder
(γ) mit 2-Diazo-1,3-diketonen der allgemeinen Formel (IX) 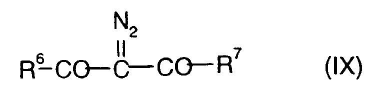 in welcher
   R⁶ und R⁷ die oben angegebenen Bedeutungen haben,
   gegebenenfalls in Gegenwart eines Verdünnungsmittels umsetzt, oder
(δ) mit Salicylsäure oder deren Estern der allgemeinen Formel (X) 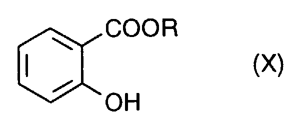 in welcher
   - R: für Wasserstoff oder Alkyl steht,
   gegebenenfalls in Gegenwart eines Säureakzeptors und gegebenenfalls in Gegenwart eines Verdünnungsmittels umsetzt (vgl. auch EP-A 371240 und die Herstellungsbeispiele).

Die als Vorprodukte benötigten Aryliso(thio)cyanate der Formel (VI) sind bekannt und/oder können nach an sich bekannten Verfahren hergestellt werden (vgl. DE-OS 2201668; DE-OS 2703838; JP-A 60/097980 - zitiert in Chem. Abstracts 103:141961; US-P 5061310; Herstellungsbeispiele).

Man erhält die Aryliso(thio)cyanate der Formel (VI), wenn man Arylamine der allgemeinen Formel (XI) 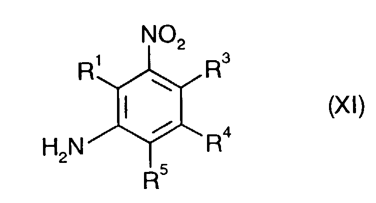 in welcher
R¹, R³, R⁴ und R⁵ die oben angegebenen Bedeutungen haben,
mit Phosgen bzw. mit Thiophosgen, gegebenenfalls in Gegenwart eines Verdünnungsmittels, wie z.B. Toluol bzw. Chloroform und Wasser, bei Temperaturen zwischen -10°C und 120°C umsetzt.

Die Vorprodukte der Formeln (VII), (VIII), (IX) und (X) sind bekannt und/oder können nach an sich bekannten Verfahren hergestellt werden (vgl. EP-A 371240; J. Heterocycl. Chem. 27, 25 (1990), J. Chem. Soc., Perkin Trans 1, 1992, 2393; Chem. Pharm. Bull. 31, 1896 (1983)).

Das erfindungsgemäße Verfahren (a) wird unter Verwendung eines Reduktionsmittels und gegebenenfalls eines Reaktionshilfsmittels durchgeführt. Es können hierbei alle für die Reduktion von Nitroverbindungen zu Aminoverbindungen üblichen Reduktionsmittel und gegebenenfalls damit zu verwendenden Reaktionshilfsmittel eingesetzt werden.

Als bevorzugte Reduktionsmittel (und gegebenenfalls damit einzusetzende Reaktionshilfsmittel) seien genannt:
Metalle, wie Eisen, Cobalt, Nickel, Zink oder Zinn in Gegenwart von Säuren, wie z.B. Essigsäure.
Wasserstoff in Gegenwart von Hydrierungskatalysatoren, wie Raney-Nickel, Raney-Cobalt, Platin oder Palladium, sowie
Metallhydridkomplexe, wie Lithiumaluminiumhydrid, Natriumborhydrid oder Kaliumborhydrid,
ferner auch Sulfide, wie Natriumsulfid oder Kaliumsulfid,
Dithionite, wie Natriumdithionit und Kaliumdithionit,
Formiate, wie Natriumformiat und Kaliumformiat.

Das erfindungsgemäße Verfahren (a) zur Herstellung der neuen Aminoaryl-1,3-oxazin-2,4-dione der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol und Isopropanol, Carbonsäuren wie Ameisensäure, Essigsäure und Propionsäure, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid, sowie Wasser.

Die Reaktionstemperaturen können bei dem erfindungsgemäßen Verfahren (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck oder unter erhöhtem oder vermindertem Druck durchgeführt (im allgemeinen zwischen 0,1 und 100 bar).

Zur Durchführung des erfindungsgemäßen Verfahrens (a) werden die jeweils benötigten Ausgangsstoffe im allgemeinen in angenähert stöchiometrischen Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).
Die beim erfindungsgemäßen Verfahren (b) zur Herstellung von Verbindungen der Formel (I) als Ausgangsstoffe zu verwendenden Aminoaryl-1,3-oxazin-2,4-dione sind durch die Formel (Ia) allgemein definiert.

In Formel (Ia) haben Q, R¹, R³, R⁴, R⁵, R⁶ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, R¹, R³, R⁴, R⁵, R⁶ und R⁷ angegeben wurden.

Die Ausgangsstoffe der Formel (Ia) für das erfindungsgemäße Verfahren (b) sind erfindungsgemäße, neue Verbindungen; sie können nach dem erfindungsgemäßen Verfahren (a) hergestellt werden.

Die beim erfindungsgemäßen Verfahren (b) weiter als Ausgangsstoffe zu verwendenden elektrophilen Verbindungen sind durch die Formel (III) bzw. (IV) allgemein definiert.

In diesen Formeln hat R² vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R² angegeben wurde;
- A¹: steht vorzugsweise für C₁-C₄-Alkyl, insbesondere für Methyl, Ethyl, n- oder i-Propyl;
- X¹: steht vorzugsweise für Chlor, Brom, Iod oder die Gruppierung -O-SO₂-O-A¹, insbesondere für Chlor, Brom, Iod, Methoxysulfonyloxy und Ethoxysulfonyloxy;
- X²: steht vorzugsweise für Fluor, Chlor, Brom, Acetoxycarbonyl oder Trifluoracetoxycarbonyl, insbesondere für Chlor oder Brom.

Die Ausgangsstoffe der Formeln (III) und (IV) sind bekannte organische Synthesechemikalien.

Die beim erfindungsgemäßen Verfahren (b) gegebenenfalls als Ausgangsstoffe zu verwendenden Isocyanate sind durch die Formel (V) allgemein definiert.

In Formel (V) steht R⁸ vorzugsweise für C₁-C₄-Alkyl, insbesondere für Methyl oder Ethyl.

Die Ausgangsstoffe der Formel (V) sind bekannte organische Synthesechemikalien.

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.
Als Säureakzeptoren können bei dem erfindungsgemäßen Verfahren (b) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calcium-hydroxid Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -20°C und + 100°C, vorzugsweise bei Temperaturen zwischen -10°C und +80°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in mono- und dikotylen Kulturen, vor allem im Nachauflauf-Verfahren.
In gewissem Umfang zeigen die Verbindungen der Formel (I) auch fungizide Wirkung, beispielsweise gegen Pyricularia oryzae am Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele

### Beispiel 1

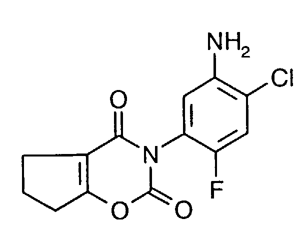

### (Verfahren (a))

Zu einem Gemisch aus 3,27 g (0,01 Mol) 3-(4-Chlor-2-fluor-5-nitrophenyl)-2,4,5,6,7-hexahydrocyclopent[e]-1,3-oxazin-2,4(3H,5H)-dion, 4,5 ml Essigsäure, 4,5 ml Wasser und 20 ml Ethylacetat gibt man bei Raumtemperatur (20°C) portionsweise 5,6 g (0,1 Mol) Eisenpulver. Man rührt noch 16 Stunden nach, fügt 20 ml Ethylacetat zu und saugt über Kieselgel ab. Nach Waschen des Filtrats mit einer gesättigten Natriumhydrogencarbonatlösung und Wasser, Trocknen über Natriumsulfat, Abfiltrieren und Einengen erhält man 2,70 g (91% der Theorie) 3-(5-Amino-4-chlor-2-fluorphenyl)-2,3,4,5,6,7-hexahydrocyclopenta[e]-1,3-oxazin-2,4-dion vom Schmelzpunkt 93°C.

¹H-NMR (80 Mhz, CDCl₃, δ): 7,19 (d, 1H, J = 9,0 Hz); 6,64 (d, 1H, J = 7,0 Hz) 4,00 (bs, 2H); 2,86 (dt, 2H, J = 8,0 Hz, J = 2 Hz); 2,76 (dt, 2H, J = 8,0 Hz, J = 2 Hz); 2,17 (quintett, 2H, J = 8,0 Hz).

### Beispiel 2

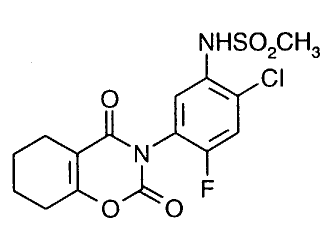

### (Verfahren (b))

Man versetzt eine Lösung von 0,3 g (0,001 Mol) 3-(5-Amino-4-chlor-2-fluorphenyl)-2,3,4,5,6,7,8-heptahydrocyclohexa[e]-1,3-oxazin-2,4-dion bei 0°C mit 0,09 g (0,0011 Mol) Pyridin und 0,13 g (0,0011 Mol) Methansulfonylchlorid und rührt über Nacht nach. Nach Aufarbeitung analog Beispiel 1 erhält man 0,34 g (88% der Theorie) 3-(5-Methylsulfonyl-4-chlor-2-fluorphenyl)-2,3,4,5,6,7,8-heptahydrocyclohexa[e]-1,3-oxazin-2,4-dion als Öl.

¹H-NMR (80 Mhz, CDCl₃, δ): 7,58 (d, 1H, J = 8,0 Hz); 7,29 (d, 1H, J = 9,0 Hz); 6,94 (bs, 1H); 2,97 (s, 3H); 2,45 (m, 2H); 2,35 (m, 4H); 1,82 (m, 2H); 1,69 (m, 2H).

### Beispiel 3

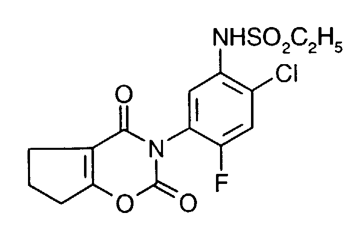

### (Verfahren (b))

Zu einer Lösung von 2,37 g (0,008 Mol) 3-(5-Amino-4-chlor-2-fluorphenyl-2,3,4,5,6,7-hexahydrocyclopenta[e]-1,3-oxazin-2,4(3H,5H)-dion in 11 ml Methylenchlorid gibt man bei 0°C 0,71 g (0,009 Mol) Pyridin und anschließend 1,13 g (0,009 Mol) Ethansulfonylchlorid. Nach Rühren über Nacht bei Raumtemperatur (20°C) gießt man das Reaktionsgemisch in 10 ml Wasser und trennt die Phasen. Man extrahiert die Wasserschicht mit Methylenchlorid und wäscht die vereinigten Methylenchloridextrakte mit Natriumhydrogencarbonatlösung und Wasser.

Durch Trocknen über Natriumsulfat und Einengen erhält man 2,73 g (88% der Theorie) 3-(5-Ethylsulfonylamino-4-chlor-2-fluorphenyl)-2,3,4,5,6,7-hexahydrocyclopenta[e]-1,3-oxazin-2,4(3H,5H)-dion vom Schmelzpunkt 166°C.
¹H-NMR (80 Mhz, CDCl₃, δ): 7,69 (d, 1H, J = 8,0 Hz); 7,35 (d, 1H, J = 9,0 Hz), 6,87 (s, 1H); 3,13 (q, 2H, J = 8,0 Hz); 2,88 (t, 2H, J = 8,0 Hz); 2,76 (t, 2H, J = 8,0 Hz); 2,19 (quintett, 2H, J = 8,0 Hz); 1,36 (t, 3H, J = 8,0 Hz).

Analog zu den Beispielen 1 bis 3 und entsprechend der allgemeinen Beschreibung der erfindungsgemäßen Herstellungsverfahren können die in der nachstehenden Tabelle 3 aufgeführten Verbindungen der Formel (I) hergestellt werden.

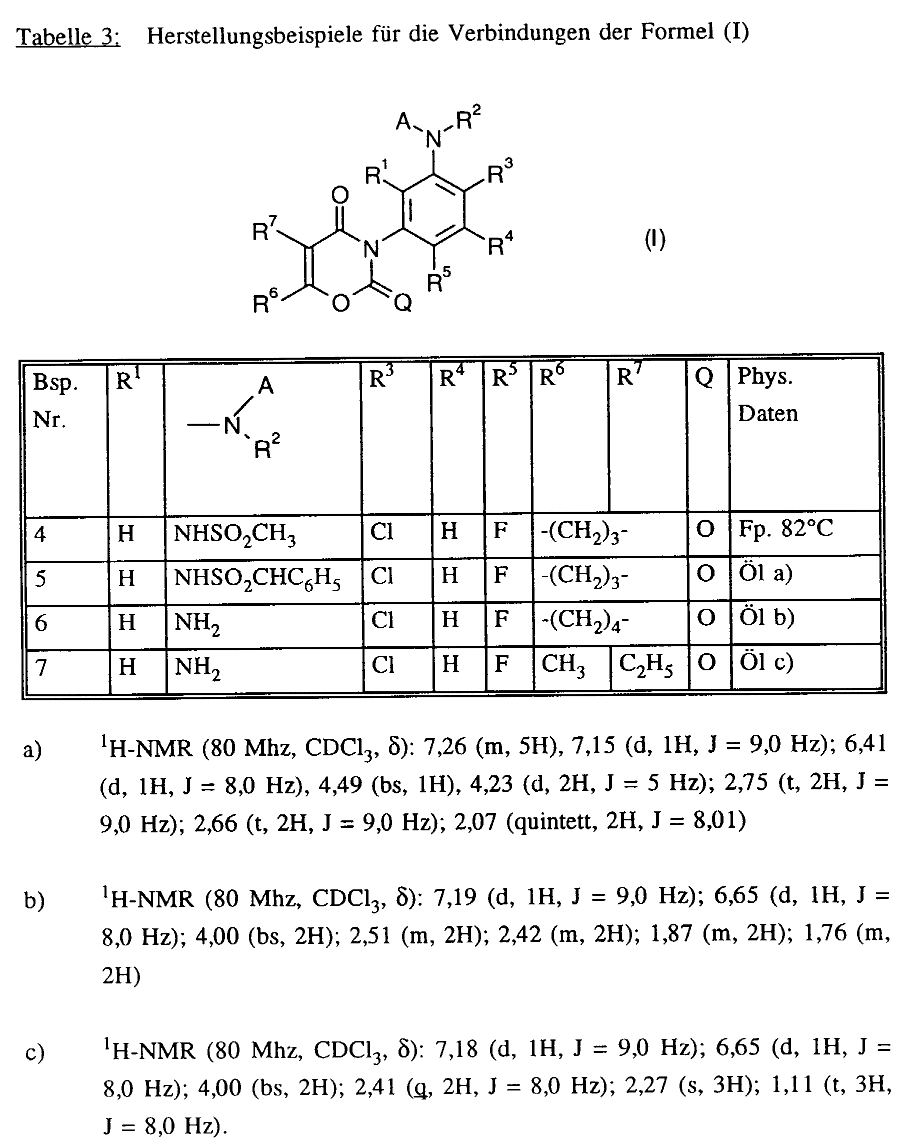

### Ausgangsstoffe der Formel (II)

### Beispiel (II-1)

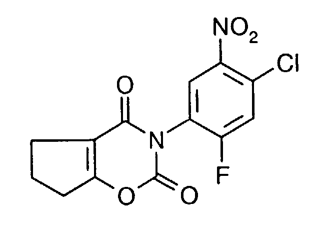

12,6 g (0,075 Mol) 2,2-Dimethyl-4,5,6,7-tetrahydropenta-1,3-dioxin-4-on werden bei 140°C zu einer Lösung von 13,5 g (0,0625 Mol) 4-Chlor-2-fluor-5-nitrophenylisocyanat in 50 ml 1,2-Dichlorbenzol tropfenweise gegeben; dann wird das Reaktionsgemisch noch 2 Stunden bei 140°C gerührt. Nach Einengen im Vakuum wird der Rückstand durch Säulenchromatographie an Kieselgel gereinigt.

Man erhält 13,4 g (66% der Theorie) 3-(4-Chlor-2-fluor-5-nitrophenyl)-2,3,4,5,6,7-hexahydropenta[e]-1,3-oxazin-2,4-dion vom Schmelzpunkt 168°C.

¹H-NMR (80 Mhz, CDCl₃, δ): 9,03 (d, 1H, J = 8,0 Hz); 7,48 (d, 1H, J = 9,0 Hz); 2,89 (m, 2H); 2,78 (m, 2H); 2,20 (quintett, 2H, J = 8,0 Hz).

### Beispiel (II-2)

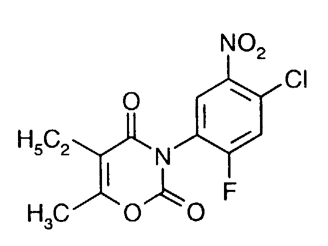

Zu einer Lösung von 5,41 g (0,025 Mol) 4-Chlor-2-fluor-5-nitro-phenylisocyanat in 20 ml o-Dichlorbenzol tropft man langsam bei 140°C 5,10 g (0,03 Mol) 5-Ethyl-2,2,6-trimethyl-4H-1,3-dioxin-4-on und erhitzt anschließend noch 2 Stunden bei dieser Temperatur. Nach Entfernen der flüchtigen Komponente isoliert man durch Säulenchromatographie über Kieselgel 3,70 g (45% der Theorie) 3,4-Dihydro-3-(4-chlor-2-fluor-5-nitrophenyl)-5-ethyl-6-methyl-2H-1,3-dion als Öl.

¹H-NMR (80 Mhz, CDCl₃, δ): 8,04 (d, 1H, J = 8,0 Hz); 7,48 (d, 1H, J = 9,0 Hz); 2,43 (q, 2H, J = 8,0 Hz); 2,31 (s, 3H); 1,12 (t, 3H, J = 8,0 Hz).

### Ausgangsstoffe der Formel (VI)

### Beispiel (VI-1)

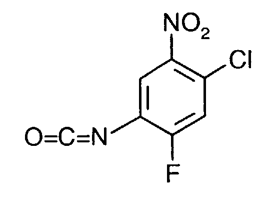

In eine Lösung von 66,7 g (0,35 Mol) 4-Chlor-2-fluor-5-nitroanilin in 1180 ml Toluol leitet man bei 0 bis 5°C trockenes HCl-Gas bis zur Sättigung ein. Anschließend dosiert man 34,7 g (0,35 ml) Phosgen zu und erhitzt langsam auf 85°C. Nach Zugabe von weiteren 17,3 g (0,175 Mol) Phosgen und Erhitzen auf 85°C (1h) bläst man den Ansatz mit Stickstoff phosgenfrei und kühlt ab. Durch Abfiltrieren vom Niederschlag und Einengen erhält man 28,6 g (37,7% der Theorie) 4-Chlor-2-fluor-5-nitrophenylisocyanat vom Schmelzpunkt 43-46°C; Siedepunkt 125°C bei 0,3 Torr (Kugelrohrdestillation).

¹H-NMR (80 Mhz, CDCl₃, δ): 7,74 (d, 1H, J = 8,0 Hz); 7,40 (d, 1H, J = 9,0 Hz). IR (Film, V): 2255, 1546, 1348 (cm⁻¹).

Analog können die in der nachstehenden Tabelle 4 aufgeführten Verbindungen der Formel (VI) hergestellt werden.

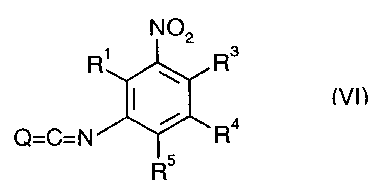

**Tabelle 4**

| Beispiele für die Verbindungen der Formel (VI) | | | | |
|---|---|---|---|---|
| R¹ | R³ | R⁴ | R⁵ | Q |
| H | Cl | H | H | O |
| H | Cl | H | F | O |
| H | Cl | H | Cl | O |
| H | Br | H | H | O |
| H | Br | H | F | O |
| H | Br | H | Cl | O |
| H | CN | H | H | O |
| H | CN | H | F | O |
| H | CN | H | Cl | O |
| H | CH₃ | H | F | O |
| H | Cl | H | F | S |
| H | CN | H | H | S |
| H | CN | H | F | S |

### Anwendungsbeispiele:

### Beispiel A

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
O % = keine Wirkung (wie unbehandelte Kontrolle)
100 % = totale Vernichtung

In diesem Test zeigen beispielsweise die Verbindungen gemäß den Herstellungsbeispielen 1, 3 und 4 bei guter Verträglichkeit gegenüber Kulturpflanzen, wie z.B. Weizen, starke Wirkung gegen Unkräuter wie Abutilon, Datura, Ipomoea Portulaca, Solanum und Xanthium, deren Schädigung jeweils zwischen 90 und 100 % liegt.

## Patentansprüche

1. Aminoaryl-1,3-oxazin-2,4-dione der allgemeinen Formel (I) 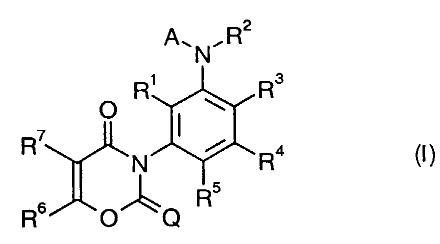 in welcher
A für Wasserstoff oder C₁-C₆-Alkyl steht,
Q für Sauerstoff oder Schwefel steht,
R¹ für Wasserstoff oder Halogen steht,
R² für Wasserstoff, für einen jeweils gegebenenfalls durch Halogen oder C₁-C₄-Alkoxy substituierten Rest der Reihe C₁-C₆-Alkyl, C₂-C₆-Alkenyl, C₂-C₆-Alkinyl, C₁-C₆-Alkyl-carbonyl, C₁-C₆-Alkoxycarbonyl, C₁-C₆-Alkylamino-carbonyl, C₁-C₆-Alkyl-sulfonyl, oder für Di-(C₁-C₄-alkyl)-amino-sulfonyl steht,
R² weiterhin für einen jeweils gegebenenfalls durch Halogen, Cyano, Nitro, Carboxy, Carbamoyl, C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) oder durch C₁-C₄-Alkoxy-carbonyl substituierten Rest der Reihe Phenyl, Phenylcarbonyl, Phenyl-C₁-C₄-alkyl, Phenylsulfonyl oder Phenyl-C₁-C₄-alkylsulfonyl steht,
R³ für Wasserstoff, Halogen, Cyano, Methyl, Trifluormethyl oder Methoxycarbonyl steht,
R⁴ für Wasserstoff oder Halogen steht,
R⁵ für Wasserstoff oder Halogen steht,
R⁶ für Wasserstoff, Halogen oder für jeweils gegebenenfalls durch Halogen substituiertes C₁-C₄-Alkyl oder Phenyl steht und
R⁷ für Wasserstoff, Halogen oder für jeweils gegebenenfalls durch Halogen substituiertes C₁-C₄-Alkyl oder Phenyl steht, oder zusammen mit R⁶ für C₃-C₅-Alkandiyl steht, oder zusammen mit R⁶ eine Benzogruppierung bildet.

2. Aminoaryl-1,3-oxazin-2,4-dione der allgemeinen Formel (I) gemäß Anspruch 1, dadurch gekennzeichnet, daß
A für Wasserstoff oder C₁-C₄-Alkyl steht,
Q für Sauerstoff oder Schwefel steht,
R¹ für Wasserstoff, Fluor, Chlor oder Brom steht,
R² für Wasserstoff, für einen jeweils gegebenenfalls durch Fluor, Chlor, Methoxy oder Ethoxy substituierten Rest der Reihe C₁-C₄-Alkyl, C₂-C₄-Alkenyl, C₂-C₄-Alkinyl, C₁-C₄-Alkyl-carbonyl, C₁-C₄-Alkoxy-carbonyl, C₁-C₄-Alkylamino-carbonyl oder C₁-C₄-Alkyl-sulfonyl oder Di-(C₁-C₃-alkyl)-amino-sulfonyl steht,
R² weiterhin für einen jeweils gegebenenfalls durch Halogen, Cyano, Nitro, Carboxy, Carbamoyl, C₁-C₃-Alkyl, C₁-C₃-Alkoxy, C₁-C₃-Alkylthio, C₁-C₃-Alkylsulfinyl, C₁-C₃-Alkylsulfonyl (welche jeweils gegebenenfalls durch Fluor und/oder Chlor substituiert sind) oder C₁-C₃-Alkoxy-carbonyl substituierten Rest der Reihe Phenyl, Phenylcarbonyl, Phenyl-C₁-C₃-alkyl, Phenylsulfonyl oder Phenyl-C₁-C₃-alkylsulfonyl steht,
R³ für Wasserstoff, Fluor, Chlor, Brom, Cyano, Methyl, Trifluormethyl oder Methoxycarbonyl steht,
R⁴ für Wasserstoff, Fluor, Chlor oder Brom steht,
R⁵ für Wasserstoff, Fluor, Chlor oder Brom steht,
R⁶ für Wasserstoff, Fluor, Chlor, Brom oder für jeweils gegebenenfalls durch Fluor und/oder Chlor substituiertes C₁-C₃-Alkyl oder Phenyl steht und
R⁷ für Wasserstoff, Fluor, Chlor, Brom oder für jeweils gegebenenfalls durch Fluor und/oder Chlor substituiertes C₁-C₃-Alkyl oder Phenyl steht, oder zusammen mit R⁶ für C₃-C₄-Alkandiyl steht, oder zusammen mit R⁶ eine Benzogruppierung bildet.

3. Aminoaryl-1,3-oxazin-2,4-dione der allgemeinen Formel (I) gemäß Anspruch 1, dadurch gekennzeichnet, daß
A für Wasserstoff, Methyl, Ethyl, und/oder i-Propyl steht,
Q für Sauerstoff oder Schwefel steht,
R¹ für Wasserstoff steht,
R² für Wasserstoff, Methyl, Ethyl, n- oder i-Propyl, Allyl, Propargyl, für jeweils gegebenenfalls durch Fluor oder Chlor substituiertes Acetyl oder Propionyl, für Methoxycarbonyl, Ethoxycarbonyl, Methylaminocarbonyl oder Ethylaminocarbonyl, für jeweils gegebenenfalls durch Fluor oder Chlor substituiertes Methylsulfonyl oder Ethylsulfonyl, für Dimethylaminosulfonyl, für jeweils gegebenenfalls durch Fluor, Chlor, Cyano, Nitro, Carboxy, Methyl, Trifluormethyl, Methoxy oder Methoxycarbonyl substituiertes Phenyl, Benzoyl, Benzyl, Phenylsulfonyl oder Phenylmethylsulfonyl steht,
R³ für Chlor, Brom oder Cyano steht,
R⁴ für Wasserstoff steht,
R⁵ für Wasserstoff, Chlor oder Fluor steht,
R⁶ für Wasserstoff, Chlor, Brom, Methyl oder Trifluormethyl steht und
R⁷ für Wasserstoff, Chlor, Brom, Methyl oder Trifluormethyl steht, oder
zusammen mit R⁶ für Trimethylen oder Tetramethylen steht.

4. Verfahren zur Herstellung von Aminoaryl-1,3-oxazin-2,4-dionen der allgemeinen Formel (I) 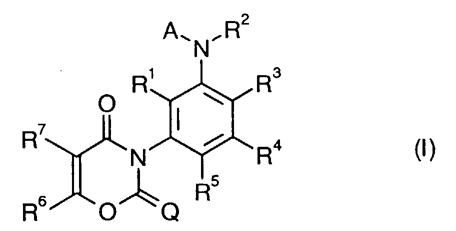 in welcher
A für Wasserstoff oder C₁-C₆-Alkyl steht,
Q für Sauerstoff oder Schwefel steht,
R¹ für Wasserstoff oder Halogen steht,
R² für Wasserstoff, für einen jeweils gegebenenfalls durch Halogen oder C₁-C₄-Alkoxy substituierten Rest der Reihe C₁-C₆-Alkyl, C₂-C₆-Alkenyl, C₂-C₆-Alkinyl, C₁-C₆-Alkyl-carbonyl, C₁-C₆-Alkoxy-carbonyl, C₁-C₆-Alkylamino-carbonyl, -carbonyl, C₁-C₆-Alkyl-sulfonyl oder für Di-(C₁-C₄-alkyl)-amino-sulfonyl steht,
R² weiterhin für einen jeweils gegebenenfalls durch Halogen, Cyano, Nitro, Carboxy, Carbamoyl, C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) oder durch C₁-C₄-Alkoxy-carbonyl substituierten Rest der Reihe Phenyl, Phenylcarbonyl, Phenyl-C₁-C₄-alkyl, Phenylsulfonyl oder Phenyl-C₁-C₄-alkylsulfonyl steht,
R³ für Wasserstoff, Halogen, Cyano, Methyl, Trifluormethyl oder Methoxycarbonyl steht,
R⁴ für Wasserstoff oder Halogen steht,
R⁵ für Wasserstoff oder Halogen steht,
R⁶ für Wasserstoff, Halogen oder für jeweils gegebenenfalls durch Halogen substituiertes C₁-C₄-Alkyl oder Phenyl steht und
R⁷ für Wasserstoff, Halogen oder für jeweils gegebenenfalls durch Halogen substituiertes C₁-C₄-Alkyl oder Phenyl steht oder zusammen mit R⁶ für C₃-C₅-Alkandiyl steht, oder zusammen mit R⁶ eine Benzogruppierung bildet,
dadurch gekennzeichnet, daß
(a) Nitroaryl-1,3-oxazin-2,4-dione der allgemeinen Formel (II) 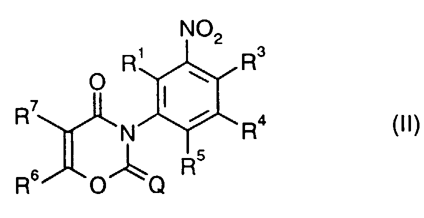 in welcher
Q, R¹, R³, R⁴, R⁵, R⁶ und R⁷ die oben angegebenen Bedeutungen haben,
mit einem Reduktionsmittel, gegebenenfalls in Gegenwart eines Katalysators oder Reaktionshilfsmittels und gegebenenfalls in Gegenwart eines Verdünnungsmittels umsetzt, oder daß man
(b) Aminoaryl-1,3-oxazin-2,4-dione der allgemeinen Formel (Ia) 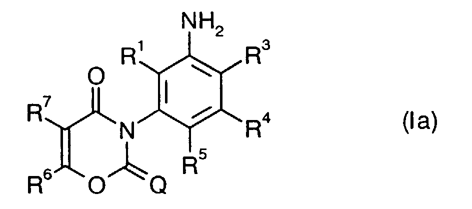 in welcher
Q, R¹, R³, R⁴, R⁵, R⁶ und R⁷ die oben angegebenen Bedeutungen haben,
mit elektrophilen Verbindungen der allgemeinen Formel (III)
X¹-A¹ (III)
in welcher
A¹ für C₁-C₆-Alkyl steht und
X¹ für Halogen oder die Gruppierung -O-SO₂-O-A¹ steht,
und/oder mit elektrophilen Verbindungen der allgemeinen Formel (IV)
X²-R² (IV)
in welcher
R² mit Ausnahme von Wasserstoff die oben angegebene Bedeutung hat und
X² für Halogen oder die Gruppierung 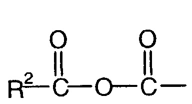 steht,
und/oder gegebenenfalls mit Isocyanaten der allgemeinen Formel (V)
O=C=N-R⁸ (V)
in welcher
R⁸ für C₁-C₆-Alkyl steht,
gegebenenfalls in Gegenwart eines Säureakzeptors und gegebenenfalls in Gegenwart eines Verdünnungsmittels umsetzt, wobei gegebenenfalls die Reihenfolge der Umsetzung mit den Verbindungen der Formeln (III) und/oder (IV) und/oder (V) auch anders als oben angegeben sein kann.

5. Nitroaryl-1,3-oxazin-2,4-dione der allgemeinen Formel (II) 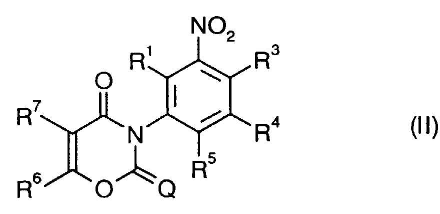 in welcher
Q für Sauerstoff oder Schwefel steht,
R¹ für Wasserstoff oder Halogen steht,
R³ für Wasserstoff, Halogen, Cyano, Methyl, Trifluormethyl oder Methoxycarbonyl steht,
R⁴ für Wasserstoff oder Halogen steht,
R⁵ für Wasserstoff oder Halogen steht,
R⁶ für Wasserstoff, Halogen oder für jeweils gegebenenfalls durch Halogen substituiertes C₁-C₄-Alkyl oder Phenyl steht und
R⁷ für Wasserstoff, Halogen oder für jeweils gegebenenfalls durch Halogen substituiertes C₁-C₄-Alkyl oder Phenyl steht, oder zusammen mit R⁶ für C₃-C₅-Alkandiyl steht, oder zusammen mit R⁶ eine Benzogruppierung bildet.

6. Amino-1,3-oxazin-2,4-dione der allgemeinen Formel (I a) 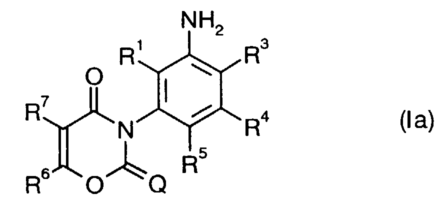 in welcher
Q für Sauerstoff oder Schwefel steht,
R¹ für Wasserstoff oder Halogen steht,
R³ für Wasserstoff, Halogen, Cyano, Methyl, Trifluormethyl oder Methoxycarbonyl steht,
R⁴ für Wasserstoff oder Halogen steht,
R⁵ für Wasserstoff oder Halogen steht,
R⁶ für Wasserstoff, Halogen oder für jeweils gegebenenfalls durch Halogen substituiertes C₁-C₄-Alkyl oder Phenyl steht und
R⁷ für Wasserstoff, Halogen oder für jeweils gegebenenfalls durch Halogen substituiertes C₁-C₄-Alkyl oder Phenyl steht, oder zusammen mit R⁶ für C₃-C₅-Alkandiyl steht, oder zusammen mit R⁶ eine Benzogruppierung bildet.

7. Herbizide Mittel, gekennzeichnet durch einen Gehalt an mindestens einem Aminoaryl-1,3-oxazin-2,4-dion der Formel (I) gemäß den Ansprüchen 1 bis 4.

8. Verfahren zur Bekämpfung von unerwünschten Pflanzen, dadurch gekennzeichnet, daß man Aminoaryl-1,3-oxazin-2,4-dione der allgemeinen Formel (I) gemäß den Ansprüchen 1 bis 4 auf Pflanzen und/oder ihren Lebensraum einwirken läßt.

9. Verwendung von Aminoaryl-1,3-oxazin-2,4-dionen der allgemeinen Formel (I) gemäß den Ansprüchen 1 bis 4 zur Bekämpfung von unerwünschten Pflanzen.

10. Verfahren zur Herstellung von herbiziden Mitteln, dadurch gekennzeichnet, daß man Aminoaryl-1,3-oxazin-2,4-dione der allgemeinen Formel (I) gemäß den Ansprüchen 1 bis 4 mit Streckmitteln und/oder oberflächenaktiven Substanzen vermischt.

## Claims

1. Aminoaryl-1,3-oxazine-2,4-diones of the general formula (I) 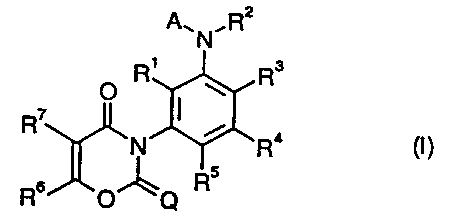 in which
A represents hydrogen or C₁-C₆-alkyl,
Q represents oxygen or sulphur,
R¹ represents hydrogen or halogen,
R² represents hydrogen, a radical from the series comprising C₁-C₆-alkyl, C₂-C₆-alkenyl, C₂-C₆-alkinyl, C₁-C₆-alkylcarbonyl, C₁-C₆-alkoxycarbonyl, C₁-C₆-alkylaminocarbonyl and C₁-C₆-alkylsulphonyl, each of which is optionally substituted by halogen or C₁-C₄-alkoxy, or represents di-(C₁-C₄-alkyl)-aminosulphonyl,
R² furthermore represents a radical from the series comprising phenyl, phenylcarbonyl, phenyl-C₁-C₄-alkyl, phenylsulphonyl or phenyl-C₁-C₄-alkylsulphonyl, each of which is optionally substituted by halogen, cyano, nitro, carboxyl, carbamoyl, C₁-C₄-alkyl, C₁-C₄-alkoxy, C₁-C₄-alkylthio, C₁-C₄-alkylsulphinyl or C₁-C₄-alkylsulphonyl (each of which is optionally substituted by halogen) or by C₁-C₄-alkoxycarbonyl,
R³ represents hydrogen, halogen, cyano, methyl, trifluoromethyl or methoxycarbonyl,
R⁴ represents hydrogen or halogen,
R⁵ represents hydrogen or halogen,
R⁶ represents hydrogen, halogen, or represents C₁-C₄-alkyl or phenyl, each of which is optionally substituted by halogen, and
R⁷ represents hydrogen, halogen, or represents C₁-C₄-alkyl or phenyl, each of which is optionally substituted by halogen, or together with R⁶ represents C₃-C₅-alkanediyl, or together with R⁶ forms a benzo group.

2. Aminoaryl-1,3-oxazine-2,4-diones of the general formula (I) according to Claim 1, characterized in that
A represents hydrogen or C₁-C₄-alkyl,
Q represents oxygen or sulphur,
R¹ represents hydrogen, fluorine, chlorine or bromine,
R² represents hydrogen, a radical from the series comprising C₁-C₄-alkyl, C₂-C₄-alkenyl, C₂-C₄-alkinyl, C₁-C₄-alkylcarbonyl, C₁-C₄-alkoxycarbonyl, C₁-C₄-alkylaminocarbonyl or C₁-C₄-alkylsulphonyl or di(C₁-C₃-alkyl)aminosulphonyl, each of which is optionally substituted by fluorine, chlorine, methoxy or ethoxy,
R² furthermore represents a radical from the series comprising phenyl, phenylcarbonyl, phenyl-C₁-C₃-alkyl, phenylsulphonyl or phenyl-C₁-C₃-alkylsulphonyl, each of which is optionally substituted by halogen, cyano, nitro, carboxyl, carbamoyl, C₁-C₃-alkyl, C₁-C₃-alkoxy, C₁-C₃-alkylthio, C₁-C₃-alkylsulphinyl, C₁-C₃-alkylsulphonyl (each of which is optionally substituted by fluorine and/or chlorine) or C₁-C₃-alkoxycarbonyl,
R³ represents hydrogen, fluorine, chlorine, bromine, cyano, methyl, trifluoromethyl or methoxycarbonyl,
R⁴ represents hydrogen, fluorine, chlorine or bromine,
R⁵ represents hydrogen, fluorine, chlorine or bromine,
R⁶ represents hydrogen, fluorine, chlorine, bromine, or represents C₁-C₃-alkyl or phenyl, each of which is optionally substituted by fluorine and/or chlorine, and
R⁷ represents hydrogen, fluorine, chlorine, bromine, or represents C₁-C₃-alkyl or phenyl, each of which is optionally substituted by fluorine and/or chlorine, or
together with R⁶ represents C₃-C₄-alkanediyl, or
together with R⁶ forms a benzo group.

3. Aminoaryl-1,3-oxazine-2,4-diones of the general formula (I) according to Claim 1, characterized in that
A represents hydrogen, methyl, ethyl and/or i-propyl,
Q represents oxygen or sulphur,
R¹ represents hydrogen,
R² represents hydrogen, methyl, ethyl, n- or i-propyl, allyl, propargyl, or represents acetyl or propionyl, each of which is optionally substituted by fluorine or chlorine, or represents methoxycarbonyl, ethoxycarbonyl, methylaminocarbonyl or ethylaminocarbonyl, or represents methylsulphonyl or ethylsulphonyl, each of which is optionally substituted by fluorine or chlorine, or represents dimethylaminosulphonyl, or represents phenyl, benzoyl, benzyl, phenylsulphonyl or phenylmethylsulphonyl, each of which is optionally substituted by fluorine, chlorine, cyano, nitro, carboxyl, methyl, trifluoromethyl, methoxy or methoxycarbonyl,
R³ represents chlorine, bromine or cyano,
R⁴ represents hydrogen,
R⁵ represents hydrogen, chlorine or fluorine,
R⁶ represents hydrogen, chlorine, bromine, methyl or trifluoromethyl and
R⁷ represents hydrogen, chlorine, bromine, methyl or trifluoromethyl, or together with R⁶ represents trimethylene or tetramethylene.

4. Process for the preparation of aminoaryl-1,3-oxazine-2,4-diones of the general formula (I) 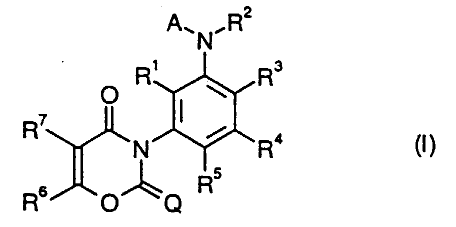 in which
A represents hydrogen or C₁-C₆-alkyl,
Q represents oxygen or sulphur,
R¹ represents hydrogen or halogen,
R² represents hydrogen, a radical from the series comprising C₁-C₆-alkyl, C₂-C₆-alkenyl, C₂-C₆-alkinyl, C₁-C₆-alkylcarbonyl, C₁-C₆-alkoxycarbonyl, C₁-C₆-alkylaminocarbonyl and C₁-C₆-alkylsulphonyl, each of which is optionally substituted by halogen or C₁-C₄-alkoxy, or represents di-(C₁-C₄-alkyl)-aminosulphonyl,
R² furthermore represents a radical from the series comprising phenyl, phenylcarbonyl, phenyl-C₁-C₄-alkyl, phenylsulphonyl or phenyl-C₁-C₄-alkyl-sulphonyl, each of which is optionally substituted by halogen, cyano, nitro, carboxyl, carbamoyl, C₁-C₄-alkyl, C₁-C₄-alkoxy, C₁-C₄-alkylthio, C₁-C₄-alkylsulphinyl or C₁-C₄-alkylsulphonyl (each of which is optionally substituted by halogen) or by C₁-C₄-alkoxycarbonyl,
R³ represents hydrogen, halogen, cyano, methyl, trifluoromethyl or methoxycarbonyl,
R⁴ represents hydrogen or halogen,
R⁵ represents hydrogen or halogen,
R⁶ represents hydrogen, halogen, or represents C₁-C₄-alkyl or phenyl, each of which is optionally substituted by halogen, and
R⁷ represents hydrogen, halogen, or represents C₁-C₄-alkyl or phenyl, each of which is optionally substituted by halogen, or together with R⁶ represents C₃-C₅-alkanediyl, or together with R⁶ forms a benzo group,
characterized in that
(a) nitroaryl-1,3-oxazine-2,4-diones of the general formula (II) 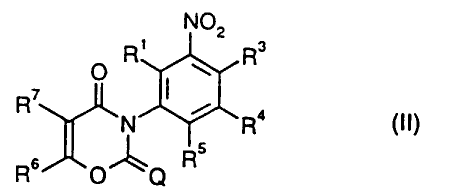 in which
Q, R¹, R³, R⁴, R⁵, R⁶ and R⁷ have the abovementioned meanings,
are reacted with a reducing agent, if appropriate in the presence of a catalyst or reaction auxiliary and if appropriate in the presence of a diluent, or in that
(b) aminoaryl-1,3-oxazine-2,4-diones of the general formula (Ia) 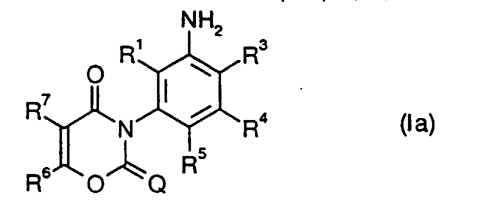 in which
Q, R¹, R³, R⁴, R⁵, R⁶ and R⁷ have the abovementioned meanings,
are reacted with electrophilic compounds of the general formula (III)
X¹-A¹ (III)
in which
A¹ represents C₁-C₆-alkyl and
X¹ represents halogen or the group -O-SO₂-O-A¹,
and/or with electrophilic compounds of the general formula (IV)
X²-R² (IV)
in which
R² has the abovementioned meaning, with the exception of hydrogen, and
X² represents halogen or the group
and/or, if appropriate, with isocyanates of the general formula (V)
O=C=N-R⁸ (V)
in which
R⁸ represents C₁-C₆-alkyl,
if appropriate in the presence of an acid acceptor and if appropriate in the presence of a diluent, it being possible, if appropriate, for the sequence of the reaction with the compounds of the formulae (III) and/or (IV) and/or (V) to differ from the sequence indicated above.

5. Nitroaryl-1,3-oxazine-2,4-diones of the general formula (II) 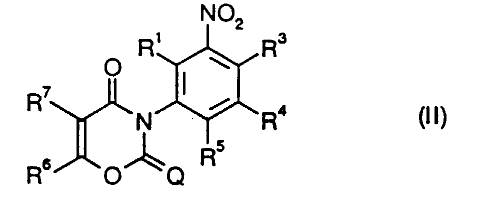 in which
Q represents oxygen or sulphur,
R¹ represents hydrogen or halogen,
R³ represents hydrogen, halogen, cyano, methyl, trifluoromethyl or methoxycarbonyl,
R⁴ represents hydrogen or halogen,
R⁵ represents hydrogen or halogen,
R⁶ represents hydrogen, halogen, or represents C₁-C₄-alkyl or phenyl, each of which is optionally substituted by halogen, and
R⁷ represents hydrogen, halogen, or represents C₁-C₄-alkyl or phenyl, each of which is optionally substituted by halogen, or together with R⁶ represents C₃-C₅-alkanediyl, or together with R⁶ forms a benzo group.

6. Amino-1,3-oxazine-2,4-diones of the general formula (I a) 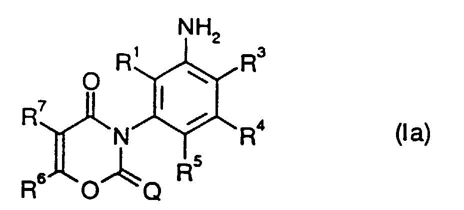 in which
Q represents oxygen or sulphur,
R¹ represents hydrogen or halogen,
R³ represents hydrogen, halogen, cyano, methyl, trifluoromethyl or methoxycarbonyl,
R⁴ represents hydrogen or halogen,
R⁵ represents hydrogen or halogen,
R⁶ represents hydrogen, halogen, or represents C₁-C₄-alkyl or phenyl, each of which is optionally substituted by halogen, and
R⁷ represents hydrogen, halogen, or represents C₁-C₄-alkyl or phenyl, each of which is optionally substituted by halogen, or together with R⁶ represents C₃-C₅-alkanediyl, or together with R⁶ forms a benzo group.

7. Herbicidal compositions, characterized in that they contain at least one aminoaryl-1,3-oxazine-2,4-dione of the formula (I) according to Claims 1 to 4.

8. Method of combating undesirable plants, characterized in that aminoaryl-1,3-oxazine-2,4-diones of the general formula (I) according to Claims 1 to 4 are allowed to act on plants and/or their environment.

9. Use of aminoaryl-1,3-oxazine-2,4-diones of the general formula (I) according to Claims 1 to 4 for combating undesired plants.

10. Process for the preparation of herbicidal compositions, characterized in that aminoaryl-1,3-oxazine-2,4-diones of the general formula (I) according to Claims 1 to 4 are mixed with extenders and/or surface-active substances.

## Revendications

1. Amino-aryl-1,3-oxazine-2,4-diones de formule générale (I) 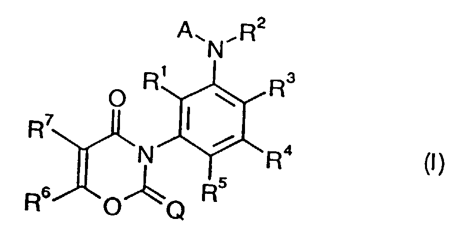 dans laquelle
A représente de l'hydrogène ou un groupe alkyle en C₁ à C₆,
Q est de l'oxygène ou du soufre,
R¹ est de l'hydrogène ou un halogène,
R² représente de l'hydrogène, un reste de la série alkyle en C₁ à C₆, alcényle en C₂ à C₆, alcynyle en C₂ à C₆, (alkyle en C₁ à C₄)carbonyle, (alkoxy en C₁ à C₆)carbonyle, (alkylamino en C₁ à C₆)carbonyre, ou alkylsulfonyle en C₁ à C₆, (chacun étant éventuellement substitué par du fluor, du chlore, un radical méthoxy ou éthoxy) ou un reste di-(alkyle en C₁ à C₄)-aminosulfonyle,
R² représente en outre un reste de la série phényle, phénylcarbonyle, phényl-(alkyle en C₁ à C₄), phénylsulfonyle, ou phényl-(alkylsulfonyle en C₁ à C₄), chacun étant éventuellement substitué par un radical halogéno, cyano, nitro, carboxy, carbamoyle, alkyle en C₁ à C₄, alkoxy en C₁ à C₄, alkylthio en C₁ à C₄, alkylsulfinyle en C₁ à C₄ ou alkylsulfonyle en C₁ à C₄ (dont chacun est éventuellement substitué par un halogène) ou par un groupe (alkoxy en C₁ à C₄) carbonyle,
R³ représente de l'hydrogène, un halogène, un groupe cyano, méthyle, trifluorométhyle ou méthoxycarbonyle,
R⁴ est de l'hydrogène ou un halogène,
R⁵ est de l'hydrogène ou un halogène,
R⁶ est de l'hydrogène, un halogène ou bien un groupe alkyle en C₁ à C₄ ou un groupe phényle dont chacun est éventuellement substitué par un halogène, et
R⁷ est de l'hydrogène, un halogène ou bien un groupe alkyle en C₁ à C₄ ou phényle dont chacun est éventuellement substitué par un halogène, ou forme conjointement avec R⁶ un groupe alcanediyle en C₃ à C₅, ou conjointement avec R⁶ un groupement benzo.

2. Amino-aryl-1,3-oxazine-2,4-diones de formule générale (I) suivant la revendication 1, caractérisées en ce que
A est de l'hydrogène ou un groupe alkyle en C₁ à C₄,
Q est de l'oxygène ou du soufre,
R¹ est de l'hydrogène, du fluor, du chlore ou du brome,
R² représente l'hydrogène un reste de la série alkyle en C₁ à C₆, alcényle en C₂ à C₄, alcynyle en C₂ à C₄, (alkyle en C₁ à C₄)carbonyle, (alkoxy en C₁ à C₄)carbonyle, (alkylamino en C₁ à C₄)-carbonyle, alkylsulfonyle en C₁ à C₄, chacun étant éventuellement substitué par un radical halogéno ou alkoxy en C₁ à C₄, ou un reste di-(alkyle en C₁ à C₃)-aminosulfonyle,
R² représente en outre un reste de la série phényle, phénylcarbonyle, phényl-(alkyle en C₁ à C₃), phénylsulfonyle ou phényl- (alkylsulfonyle en C₁ à C₃) dont chacun étant substitué, le cas échéant, par un radical halogéno, cyano, nitro, carboxy, carbamoyle, alkyle en C₁ à C₃, alkoxy en C₁ à C₃, alkylthio en C₁ à C₃, alkylsulfinyle en C₁ à C₃, alkylsulfonyle en C₁ à C₃ (chacun étant éventuellement substitué par du chlore et/ou du brome) ou un reste phényl-(alkylsulfonyle en C₁ à C₃),
R³ représente de l'hydrogène, du fluor, du chlore, du brome, un groupe cyano, méthyle, trifluorométhyle ou méthoxycarbonyle,
R⁴ est de l'hydrogène, du fluor, du chlore ou du brome,
R⁵ est de l'hydrogène, du fluor, du chlore ou du brome,
R⁶ est de l'hydrogène, du fluor, du chlore ou du brome ou bien un groupe alkyle en C₁ à C₃ ou un groupe phényle dont chacun est éventuellement substitué par du fluor et/ou du chlore, et
R⁷ est de l'hydrogène, du fluor, du chlore, du brome ou bien un groupe alkyle en C₁ à C₃ ou un groupe phényle dont chacun est éventuellement substitué par du fluor et/ou du chlore, ou forme conjointement avec R⁶ un groupe alcanediyle, ou conjointement avec R⁶, un groupement benzo.

3. Amino-aryl-1,3-oxazine-2,4-diones de formule générale (I) suivant la revendication 1, caractérisées en ce que
A est de l'hydrogène, un groupe méthyle, éthyle et/ou isopropyle,
Q est de l'oxygène ou du soufre,
R¹ est de l'hydrogène,
R² est de l'hydrogène, un groupe méthyle, éthyle, n-propyle, isopropyle, allyle, propargyle, un groupe acétyle ou un groupe propionyle dont chacun est éventuellement substitué par du fluor ou du chlore, un groupe méthoxycarbonyle, éthoxycarbonyle, méthylaminocarbonyle ou éthylaminocarbonyle, un groupe méthylsulfonyle ou un groupe éthylsulfonyle dont chacun est éventuellement substitué par du fluor ou du chlore, un groupe diméthylaminosulfonyle, un groupe phényle, benzoyle, benzyle, phénylsulfonyle ou phénylméthylsulfonyle dont chacun est éventuellement substitué par du fluor, du chlore, un radical cyano, nitro, carboxy, méthyle, trifluorométhyle, méthoxy ou méthoxycarbonyle,
R³ est du chlore, du brome ou un groupe cyano,
R⁴ est de l'hydrogène,
R⁵ est de l'hydrogène, du chlore ou du fluor,
R⁶ est de l'hydrogène, du chlore, du brome, un groupe méthyle ou trifluorométhyle, et
R⁷ est de l'hydrogène, du chlore, du brome, un groupe méthyle ou trifluorométhyle, ou forme conjointement avec R⁶ un groupe triméthylène ou tétraméthylène.

4. Procédé de production d'amino-aryl-1,3-oxazine-2,4-diones de formule générale (I) 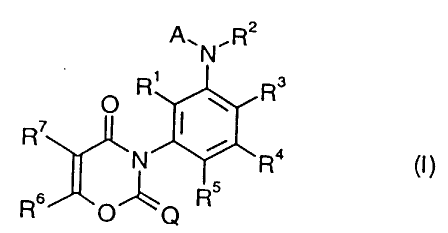 dans laquelle
A représente de l'hydrogène ou un groupe alkyle en C₁ à C₆,
Q est de l'oxygène ou du soufre,
R¹ est de l'hydrogène ou un halogène,
R² représente de l'hydrogène, un reste de la série alkyle en C₁ à C₆, alcényle en C₂ à C₆, alcynyle en C₂ à C₆, (alkyle en C₁ à C₆)carbonyle, (alkoxy en C₁ à C₆)carbonyle, (alkylamino en C₁ à C₆)-carbonyle, alkylsulfonyle en C₁ à C₆, chacun étant éventuellement substitué par un radical halogéno ou alkoxy en C₁ à C₄, ou un reste di-(alkyle en C₁ à C₄)-aminosulfonyle,
R² représente en outre un reste de la série phényle, phénylcarbonyle, phényl-(alkyle en C₁ à C₄), phénylsulfonyle, ou phényl-(alkylsulfonyle en C₁ à C₄), chacun étant éventuellement substitué par un radical halogéno, cyano, nitro, carboxy, carbamoyle, alkyle en C₁ à C₄, alkoxy en C₁ à C₄, alkylthio en C₁ à C₄, alkylsulfinyle en C₁ à C₄ ou alkylsulfonyle en C₁ à C₄ (dont chacun est éventuellement substitué par un halogène) ou par un groupe (alkoxy en C₁ à C₄)carbonyle,
R³ représente de l'hydrogène, un halogène, un groupe cyano, méthyle, trifluorométhyle ou méthoxycarbonyle,
R⁴ est de l'hydrogène ou un halogène,
R⁵ est de l'hydrogène ou un halogène,
R⁶ est de l'hydrogène, un halogène ou bien un groupe alkyle en C₁ à C₄ ou un groupe phényle dont chacun est éventuellement substitué par un halogène, et
R⁷ est de l'hydrogène, un halogène ou bien un groupe alkyle en C₁ à C₄ ou phényle dont chacun est éventuellement substitué par un halogène, ou forme conjointement avec R⁶ un groupe alcanediyle en C₃ à C₅, ou conjointement avec R⁶ un groupement benzo,
caractérisé en ce que :
(a) on fait réagir des nitro-aryl-1,3-oxazine-2,4-diones de formule générale (II) 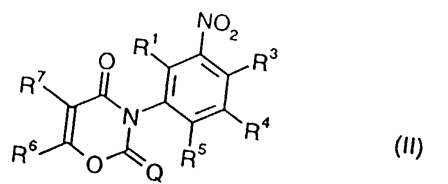 dans laquelle
Q, R¹, R³, R⁴, R⁵, R⁶ et R⁷ ont les définitions indiquées ci-dessus,
avec un agent réducteur, éventuellement en présence d'un catalyseur ou d'un auxiliaire de réaction et, le cas échéant en présence d'un diluant, ou en ce que
(b) on fait réagir des amino-aryl-1,3-oxazine-2,4-diones de formule générale (Ia) 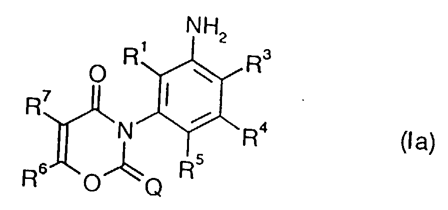 dans laquelle
Q, R¹, R³, R⁴, R⁵, R⁶ et R⁷ ont les définitions indiquées ci-dessus,
avec des composés électrophiles de formule générale (III)
X¹-A¹ (III)
dans laquelle
A¹ représente un groupe alkyle en C₁ à C₆ et
X¹ est un halogène ou le groupement -O-SO₂-O-A¹,
et/ou avec des composés électrophiles de formule générale (IV)
X²-R² (IV)
dans laquelle
R² a la définition indiquée ci-dessus, excepté l'hydrogène et
X² est un halogène ou le groupement 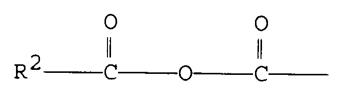
et/ou le cas échéant avec des isocyanates de formule générale (V)
O=C=N-R⁸ (V)
dans laquelle
R⁸ est un groupe alkyle en C₁ à C₆,
le cas échéant en présence d'un accepteur d'acide et la présence éventuelle d'un diluant, l'ordre de réaction avec les composés de formules (III) et/ou (IV) et/ou (V) pouvant aussi, le cas échéant, être différent de celui qui a été indiqué ci-dessus.

5. Nitro-aryl-1,3-oxazine-2,4-diones de formule générale (II) 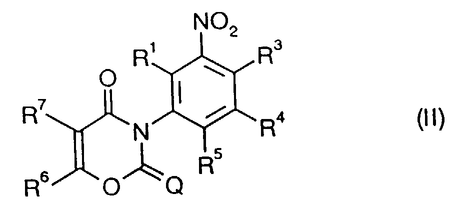 dans laquelle
Q représente de l'oxygène ou du soufre,
R¹ est de l'hydrogène ou un halogène,
R³ est de l'hydrogène, un halogène, un groupe cyano, méthyle, trifluorométhyle ou méthoxycarbonyle,
R⁴ est de l'hydrogène ou un halogène,
R⁵ est de l'hydrogène ou un halogène,
R⁶ est de l'hydrogène, un halogène ou bien un groupe alkyle en C₁ à C₄ ou un groupe phényle dont chacun est éventuellement substitué par un halogène, et
R⁷ est de l'hydrogène, un halogène ou bien un groupe alkyle en C₁ à C₄ ou un groupe phényle dont chacun est éventuellement substitué par un halogène, ou forme conjointement avec R⁶ un groupe alcanediyle en C₃ à C₅, ou conjointement avec R⁶ un groupement benzo.

6. Amino-1,3-oxazine-2,4-diones de formule générale (Ia) 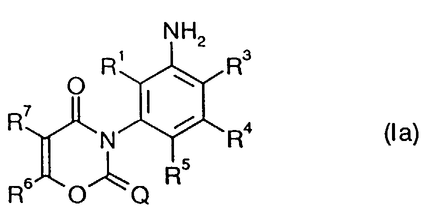 dans laquelle
Q est de l'oxygène ou du soufre,
R¹ est de l'hydrogène ou un halogène,
R³ est de l'hydrogène, un halogène, un groupe cyano, méthyle, trifluorométhyle ou méthoxycarbonyle,
R⁴ est de l'hydrogène ou un halogène,
R⁵ est de l'hydrogène ou un halogène,
R⁶ est de l'hydrogène, un halogène ou bien un groupe alkyle en C₁ à C₄ ou un groupe phényle dont chacun est éventuellement substitué par un halogène, et
R⁷ est de l'hydrogène, un halogène ou bien un groupe alkyle en C₁ à C₄ ou un groupe phényle dont chacun est éventuellement substitué par un halogène, ou forme conjointement avec R⁶ un groupe alcanediyle en C₃ à C₅, ou conjointement avec R⁶ un groupement benzo.

7. Compositions herbicides, caractérisées par une teneur en au moins une amino-aryl-1,3-oxazine-2,4-dione de formule (I) suivant les revendications 1 à 4.

8. Procédé pour combattre des plantes indésirables, caractérisé en ce qu'on fait agir des amino-aryl-1,3-oxazine-2,4-diones de formule générale (I) suivant les revendications 1 à 4 sur les plantes et/ou sur leur milieu.

9. Utilisation d'amino-aryl-1,3-oxazine-2,4-diones de formule générale (I) suivant les revendications 1 à 4 pour combattre les plantes indésirables.

10. Procédé de préparation de compositions herbicides, caractérisé en ce qu'on mélange des amino-aryl-1,3-oxazine-2,4-diones de formule générale (I) suivant les revendications 1 à 4 avec des diluants et/ou des substances tensio-actives.
